# EUROPÄISCHE PATENTANMELDUNG

(11) **EP 2 052 604 A1**
(43) Veröffentlichungstag der Anmeldung: **29.04.2009**
(21) Anmeldenummer: 07020807.9
(22) Anmeldetag: 24.10.2007
(51) Int. Cl.: A01N 47/36, C07D 251/16, C07C 311/65, A01P 13/00

(54) **Salz des 2-lodo-N-[(4-methoxy-6-methyl-1,3,5-triazin-2-yl)carbamoyl] benzolsulfonamids,Verfahren zu deren Herstellung, sowie deren Verwendung als Herbizide und Pflanzenwachstumregulatoren**

(71) Anmelder: Bayer CropScience AG, 40789 Monheim (DE)
(72) Erfinder: Waldraff, Christian, Dr., 61118 Bad Vilbel (DE); Müller, Klaus-Helmut, Dr., 40593 Düsseldorf (DE); Gesing, R. F. Ernst, Dr., 40699 Erkrath-Hochdahl (DE); Dittgen, Jan, Dr., 60316 Frankfurt (DE); Feucht, Dieter, Dr., 65760 Eschborn (DE); Rosinger, Chris, Dr., 65719 Hofheim (DE)

(57) **Zusammenfassung**

Diese Erfindung betrifft Salze des 2-Iodo-N-[(4-methoxy-6-methyl-1,3,5-triazin-2-yl)carbamoyl]benzolsulfonamids, Verfahren zu deren Herstellung, sowie deren Verwendung als Herbizide, insbesondere als Herbizide zur selektiven Bekämpfung unerwünschter Schadpflanzen in Nutzpflanzenkulturen Dauerkulturen oder Nichtkulturland, sowie als Pflanzenwachstumsregulatoren allein, oder mit Safenem und/oder in Kombination mit anderen Herbiziden, deren Anwendung zur Bekämpfung unerwünschter Schadpflanzen (wie beispielsweise Unkräuter/Ungräser) in speziellen Pflanzenkulturen oder als Pflanzenschutzregulatoren bekannt ist, bei gleichzeitiger und/oder sequentieller Anwendung, entweder als Fertigformulierung oder als Tankmix.

## Beschreibung

Diese Erfindung betrifft Salze des 2-lodo-N-[(4-methoxy-6-methyl-1,3,5-triazin-2-yl)carbamoyl]benzolsulfonamids, Verfahren zu deren Herstellung, sowie deren Verwendung als Herbizide, insbesondere als Herbizide zur selektiven Bekämpfung unerwünschter Schadpflanzen in Nutzpflanzenkulturen Dauerkulturen oder Nichtkulturland, sowie als Pflanzenwachstumsregulatoren allein, oder mit Safenem und/oder in Kombination mit anderen Herbiziden, deren Anwendung zur Bekämpfung unerwünschter Schadpflanzen (wie beispielsweise Unkräuter/Ungräser) in speziellen Pflanzenkulturen oder als Pflanzenschutzregulatoren bekannt ist, bei gleichzeitiger und/oder sequentieller Anwendung, entweder als Fertigformulierung oder als Tankmix.

Es ist bekannt, daß substituierte Phenylsulfonylhamstoffe, herbizide Eigenschaften aufweisen. Dabei handelt es sich z.B. um Phenylderivate, die einfach oder mehrfach substituiert sind (z.B. US 4127405, WO 9209608, BE 853374, WO 9213845, EP 84020, WO 9406778, WO 02072560, US 4169719, US4629494, DE 4038430).

Überraschenderweise wurden nun gefunden, dass Salze des 2-lodo-N-[(4-methoxy-6-methyl-1,3,5-triazin-2-yl)carbamoyl]benzolsulfonamids, sich besonders vorteilhaft als Herbizide und/oder Pflanzenwachstumsregulatoren eignen.

Gegenstand der vorliegenden Erfindung sind somit agrochemisch wirksame Salze des 2-lodo-N-[(4-methoxy-6-methyl-1,3,5-triazin-2-yl)carbamoyl]benzolsulfonamids

Bevorzugter Gegenstand der vorliegenden Erfindung sind Verbindungen der allgemeinen Formel (I) 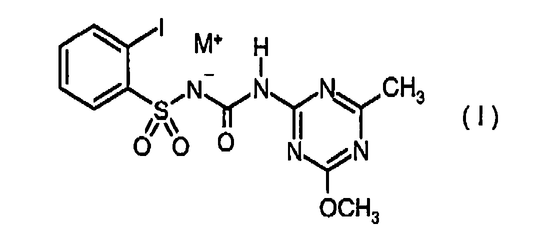 wobei
- das Kation (M⁺): (a) ein Ion der Alkalimetalle, bevorzugt Lithium, Natrium, Kalium, oder
(b) ein Ion der Erdalkalimetalle, bevorzugt Calcium und Magnesium, oder
(c) ein Ion der Übergangsmetalle, bevorzugt Mangan, Kupfer, Zink und Eisen, oder
(d) ein Ammonium-Ion, bei dem gegebenenfalls ein, zwei, drei oder alle vier Wasserstoffatome, durch gleiche oder verschiedene Reste aus der Gruppe (C₁-C₄)-Alkyl. Hydroxy-(C₁-C₄)-Alkyl, (C₃-C₆)-Cycloalkyl, (C₁-C₄)-Alkoxy-(C₁-C₄)-alkyl, Hydroxy-(C₁-C₄)-alkoxy-(C₁-C₄)-alkyl, (C₁-C₆)-Mercaptoalkyl, Phenyl oder Benzyl substituiert sind, wobei die zuvor genannten Reste gegebenenfalls durch einen oder mehrere, gleiche oder verschiedene Reste aus der Gruppe Halogen, wie F, Cl, Br oder I, Nitro, Cyano, Azido, (C₁-C₆)-Alkyl, (C₁-C₆)-Haloalkyl, (C₃-C₆)-Cycloalkyl, (C₁-C₆)-Alkoxy, (C₁-C₆)-Haloalkoxy und Phenyl substituiert sind, und wobei jeweils zwei Substituenten am N-Atom zusammen gegebenfalls einen unsubstituierten oder substituierten Ring bilden, oder
(e) ein Phosphonium-Ion, oder
(f) ein Sulfonium-Ion, bevorzugt Tri-((C₁-C₄)-alkyl)-suifonium, oder
(g) ein Oxonium-Ion, bevorzugt Tri-((C₁-C₄)-alkyl)-oxonium, oder
(h) eine gegebenenfalls einfach oder mehrfach annellierte und/oder durch (C₁-C₄)-Alkyl substituierte gesättigte oder ungesättigte/aromatische N-haltige heterocyclische ionische Verbindung mit 1-10 C-Atomen im Ringsystem ist.

Weiter bevorzugt sind Verbindungen der Formel (I), in denen das Kation (M⁺)
(a) ein Ion der Alkalimetalle, bevorzugt Lithium, Natrium, Kalium, oder
(b) ein Ion der Erdalkalimetalle, bevorzugt Calcium und Magnesium, oder
(c) ein Ion der Übergangsmetalle, bevorzugt Mangan, Kupfer, Zink und Eisen, oder
(d) ein Ammonium-Ion, bei dem gegebenenfalls ein, zwei, drei oder alle vier Wasserstoffatome, durch gleiche oder verschiedene Reste aus der Gruppe (C₁-C₄)-Alkyl, Hydroxy-(C₁-C₄)-Alkyl, (C₃-C₄)-Cycloalkyl, (C₁-C₂)-Alkoxy-(C₁-C₂)-alkyl. Hydroxy-(C₁-C₂)-alkoxy-(C₁-C₂)-alkyl, (C₁-C₂)-Mercaptoalkyl, Phenyl oder Benzyl substituiert sind, wobei die zuvor genannten Reste gegebenenfalls durch einen oder mehrere, gleiche oder verschiedene Reste aus der Gruppe Halogen, wie F, Cl, Br oder I, Nitro, Cyano, Azido, (C₁-C₂)-Alkyl, (C₁-C₂)-Haloalkyl, (C₃-C₄)-Cycloalkyl, (C₁-C₂)-Alkoxy, (C₁-C₂)-Haloalkoxy und Phenyl substituiert sind, und wobei jeweils zwei Substituenten am N-Atom zusammen gegebenfalls einen unsubstituierten oder substituierten Ring bilden, oder
(e) ein quartäres Phosphonium-Ion, bevorzugt Tetra-((C₁-C₄)-alkyl)-phosphonium und Tetraphenyl-phosponium, wobei die (C₁-C₄)-Alkylreste und die Phenylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₂)-Haloalkoxy substituiert sind, oder
(f) ein tertiäres Sulfonium-Ion, bevorzugt Ti-((C₁-C₄)-akyl)-sulfonium oder Triphenyl-sulfonium, wobei die (C₁-C₄)-Alkylreste und die Phenylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₂)-Haloalkoxy substituiert sind, oder
(g) ein tertiäres Oxonium-Ion, bevorzugt Tri-((C₁-C₄)alkyl)-oxonium, wobei die (C₁-C₄)-Alkylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₂)-Haloalkoxy substituiert sind, oder
(h) ein Kation aus der Reihe der folgenden heterocyclischen Verbindungen, wie beispielsweise Pyridin, Chinolin, 2-Methylpyridin, 3-Methylpyridin, 4-Methylpyridin, 2,4-Dimethylpyridin, 2,5-Dimethylpyridin, 2,6-Dimethylpyridin, 5-Ethyl-2-methylpyridin, Piperidin, Pyrrolidin, Morpholin, Thiomorpholin, Pyrrol, Imidazol, 1,5-Diazabicyclo[4.3.0]non-5-en (DBN), 1,8-Diazabicyclo[5.4.0]undec-7-en (DBU) ist.

Bevorzugt sind Verbindungen der Formel (I), in denen das Kation (M⁺) ein Natrium-Ion, ein Kalium-Ion, ein Lithium-Ion, ein Magnesium-Ion, ein Calcium-Ion, ein NH₄⁺-Ion, ein (2-Hydroxyeth-1-yl)ammonium-Ion, Bis-N,N-(2-hydroxyeth-1-yl)ammonium-Ion, Tris-N,N,N-(2-hydroxyeth-1-yl)ammonium-Ion, ein Methylammonium-Ion, ein Dimethylammonium-Ion, ein Trimethylammonium-Ion, ein Tetramethylammonium-Ion ein Ethylammonium-Ion, ein Diethylammonium-Ion, ein Triethylammonium-Ion, ein Tretraethylammonium-Ion ein Isopropylammonium-Ion, ein Diisopropylammonium-Ion, ein Tetrapropylammonium-Ion, ein Tetrabutylammonium-Ion, ein 2-(2-Hydroxyeth-1-oxy)eth-1-yl-ammonium-Ion, ein Di-(2-hydroxyeth-1-yl)-ammonium-Ion, ein Trimethylbenzylammonium-Ion, ein Tri-((C₁-C₄)-alkyl)-sulfonium-Ion, oder ein Tri-((C₁-C₄)-alkyl)-oxonium-Ion, ein Benzylammonium-Ion, ein 1-Phenylethylammonium-lon, ein 2-Phenylethylammonium-Ion, ein Diisopropylethylammonium-Ion, ein Pyridinium-Ion, ein Piperidinium-Ion, ein Imidazolium-Ion, ein Morpholinium-Ion, ein 1,8-Diazabicyclo[5,4.0]undec-7-enium-Ion ist.

Weiter bevorzugt sind Verbindungen der Formel (I) in denen das Kation (M⁺) ein Natrium-Ion, ein Kallum-Ion, ein Magnesium-Ion, ein Calcium-Ion, oder ein NH₄⁺-Ion ist,

Besonders bevorzugt sind Verbindungen der Formel (I), in denen das Kation (M⁺) ein Natrium-Ion, ein Kalium-Ion oder ein NH₄-Ion ist.

Ganz besonders bevorzugt sind Verbindungen der Formel (I), in denen das Kation (M⁺) ein Natrium-Ion oder ein Kalium-Ion ist.

In Formel (I) und allen nachfolgenden Formeln können die kohlenstoffhaltigen Reste wie Alkyl, Alkoxy jeweils geradkettig oder verzweigt sein z.B. Methyl, Ethyl, n- oder i-Propyl, n-, i-, t- oder 2-Butyl.

Cycloalkyl bedeutet ein carbocyclisches, gesättigtes Ringsystem mit vorzugsweise 3-6 C-Atomen, z.B. Cyclopropyl, Cyclobutyl, Cyclopentyl oder Cyclohexyl,

Gegenstand der Erfindung sind auch alle Stereoisomeren, die von erfindungsgemäßen Salzen, insbesondere von Verbindungen der Formel (I), umfasst sind, und deren Gemische. Solche erfindungsgemäßen Salze, insbesondere Verbindungen der Formel (I) enthalten ein oder mehrere asymmetrische C-Atome, die in der allgemeinen Formel (I) nicht gesondert angegeben sind. Die durch ihre spezifische Raumform definierten möglichen Stereoisomeren, wie Enantiomere oder Diastereomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl" fallen, bedeuten keine vollständige Aufzählung. Die allgemeinen Begriffe umfassen auch die weiter unten angeführten Definitionen für Restebereiche in Gruppen bevorzugter Verbindungen, insbesondere Restebereiche, welche spezifische Reste aus den Tabellenbeispielen umfassen.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Gegenstand der vorliegenden Erfindung sind auch Verfahren zur Herstellung der erfindungsgemäßen Salze, insbesondere von Vebindungen der allgemeinen Formel (I).
wobei man
a) 2-Iodbenzolsulfonamid (II) 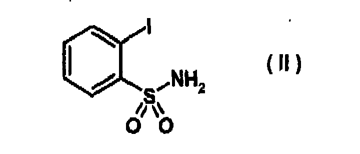 mit einem heterocyclischen Carbamat der Formel (III) 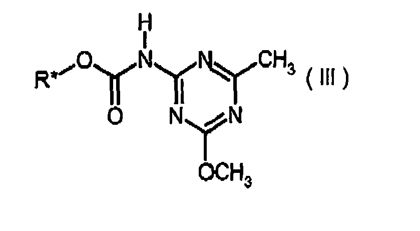 worin R* ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet,
   umsetzt, oder
b) ein Sulfonylcarbamat der Formel (IV), 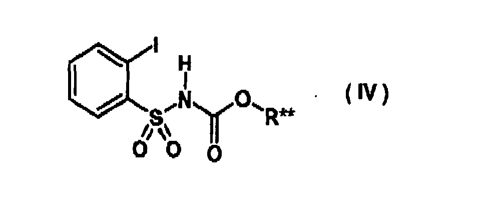 worin R** ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet,
   mit einem 2-Amino-4-methoxy-6-methyl-triazin der Formel (V) 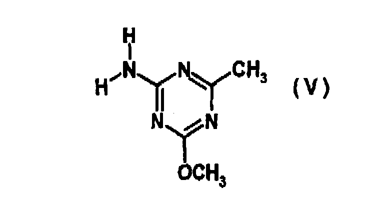 umsetzt, oder
c) 2-lodbenzolsulfonsäureisocyanat (VI) 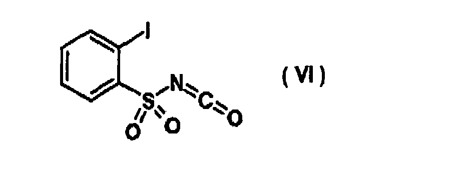 mit dem Aminoheterocyclus der Formel (V) umsetzt, oder
d) 2-Iodbenzolsulfonamid (II) mit dem Isocyanat (VII) 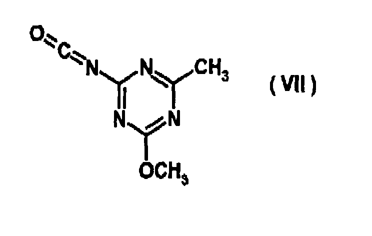 in Gegenwart einer Base umsetzt, oder
e) den Aminoheterocyclus der Formel (V) zunächst basenketalysiert mit einem Kohlensäureester, z.B. Diphenylcarbonat, umsetzt und das gebildete Intermediat in einer Eintopfreaktion mit 2-lodbenzolsulfonamid (II) (siehe Variante a)) umsetzt (vgl. JP1989221366), oder
f) ein 2-Iodbenzolsulfonsäurehalogenid der Formel (VIII), 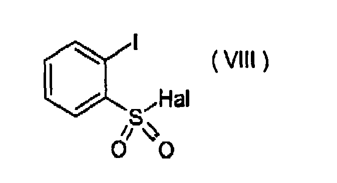 wobei Hal ein Halogenatom, vorzugsweise Chlor (VIIIa), oder Fluor (VIIIb), oder Brom (VIIIc) ist, mit einem Cyanat, beispielsweise einem Metallcyanat, insbesondere einem Alkallmetallcyanat, wie Natriumcyanat, zum Isocyanat der Formel (VI) oder einem solvatisierten (stabilisierten) Derivat davon umsetzt, und anschließend mit dem Aminoheterocyclus der Formel (V) umsetzt,
g) 2-lodbenzolsulfonamid (II) mit einem heterocyclischen Biscarbamat der Formel (IIIa), 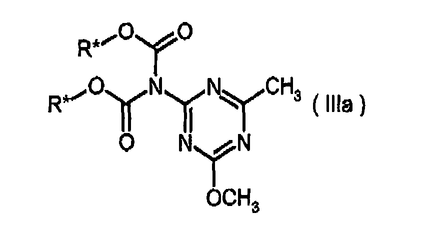 worin R* ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet, umsetzt (siehe WO 96/22284),
h) 2-lodbenzolsulfonamid (II) zunächst basenkatalysiert mit einem Kohlensäureester, z.B. Diphenylcarbamat, umsetzt und das gebildete Intermediat in einer Eintopfreaktion mit dem Aminoheterocyclus der Formel (V) (siehe Variante b)) umsetzt,

Die Umsetzung der Verbindungen der Formeln (II) und (III) gemäß Variante a) erfolgt vorzugsweise basenkatalysiert in einem inerten organischen Lösungsmittel, wie z.B. Dichlormethan, Acetonitril, Dioxan oder THF bei Temperaturen zwischen 0°C und dem Siedepunkt des Lösungsmittels, vorzugsweise bei Raumtemperatur. Als Base werden dabei beispielsweise organische Aminbasen, wie 1,8-Diazablcyclo[5.4.0]undec-7-en (DBU), Alkali-tert.-butylat, wie z.B. NaO-tert.-butylat, oder Alkalihydroxide, wie z.B. NaOH, insbesondere bei R* = (subst.) Phenyl (vgl. EP-A-44 807), oder Trialkylaluminium wie Trimethylaluminium oder Triethylaluminium, letzter insbesondere bei R* = Alkyl (vgl. EP-A-186 516) verwendet. Die jeweilige Base wird dabei beispielsweise im Bereich von 1 bis 3 Moläquivalenten, bezogen auf die Verbindung der Formel (II), eingesetzt.

2-Iodbenzolsulfonylisocyanat ist eine neue Verbindung, die ebenso wie ihre Herstellung und ihre Verwendung zur Herstellung von Verbindungen der Formel (I) Gegenstand der vorliegenden Erfindung sind.

Man kann 2-Iodbenzolsulfonamid (II) z.B. erhalten, wie in den nachfolgenden Schemata 1 bis 7 gezeigt.

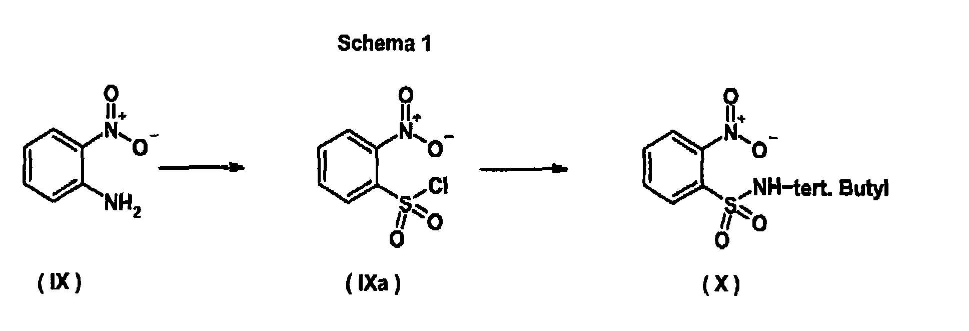

Ausgehend vom kommerziell erhältlichen 2-Nitroanilin (IX) können, z.B. durch Diazotierung der Aminogruppe mit einem Alkalinitrit, wie z. B. Natriumnitrit, in Gegenwart von Salzsäure bei Temperaturen zwischen -10°C und 10°C und nachfolgendem Austausch der resultierenden Diazogruppe z. B. mit Schwefeldioxid in Gegenwart eines Verdünnungsmittels, wie z. B. Dichlormethan, 1,2-Dichlorethan oder Essigsäure, und in Gegenwart eines Katalysators, wie z. B. Kupfer(I)chlorid und/oder Kupfer(II)chlorid, bei Temperaturen zwischen -10°C und 50°C 2-Nitrobenzolsulfonsäurechlorid (IXa) erhalten werden (vgl. Meerwein, Chem. Ber. 1957, 90, 841) (Schema 1). Alternativ zur Verwendung von Schwefeldioxid kann auch Na₂S₂O₅ (Natriummetabisulfit) als SO₂-Quelle eingesetzt werden. Durch Behandlung von (IXa) mit tert.-Butylamin kann N-tert.-Butyl-2-nitrobenzol-sulfonamid (X) erhalten werden. Die Sulfonamidbildung wird beispielsweise in inerten Lösungsmitteln wie z.B. Dichlormethan, Tetrahydrofuran (THF), Dioxan, Toluol oder Dimethylformamid (DMF) bei Temperaturen zwischen -70°C bis zum Siedepunkt des verwendeten Lösungsmittels, vorzugsweise bei 25°C durchgeführt. Dabei kommt bevorzugt eine Aminmenge von 1.5 - 2.5 Äquivalenten bezogen auf das verwendete Sulfochlorid zum Einsatz.

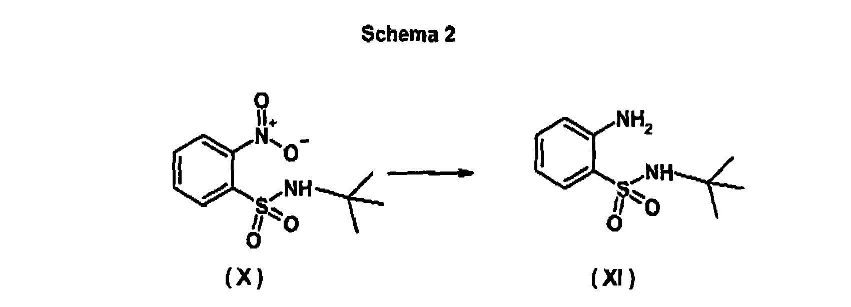

Die Reduktion von (X) zu N-tert.-Butyl-2-aminobenzolsulfonamid (XI) erfolgt analog zu bekannten Methoden (vgl. hierzu Houben-Weyl, "Methoden der Organischen Chemie", 4. Aufl. Bd. XI/1 S. 360 ff., Thieme Verlag Stuttgart, 1957) (Schema 2).

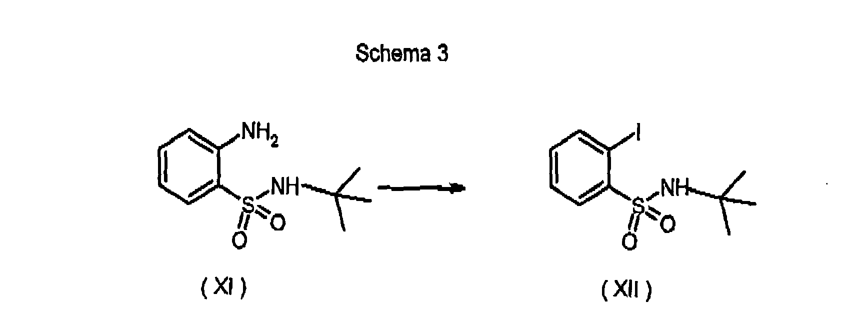

Anilin (XI) kann unter üblichen Bedingungen für Diazotierungsreaktionen diazotiert und anschließend in N-tert.-Butyl-2-iodbenzolsulfonamid (XII) überführt werden. Beispielsweise erfolgt die Diazotierung in Gegenwart der Säure H⁺X⁻, wobei X⁻ vorzugsweise Cl⁻, I⁻ der HSO₄⁻ ist, in wässriger Lösung, gegebenenfalls unter Einsatz eines unter den Reaktionsbedingungen inerten organischen Lösungsmittels mit einem Nitrit. Beispielsweise diazotiert man mit einem Alkalimetallnitrit wie NaNO₂ (Natriumnitrit) in Mengen von 1.0-1.2 Mol Nitrit, vorzugsweise 1,01 -1.05 Mol Nitrit, pro Mol Anilin (XI). Als Säuren eignen sich Mineralsäuren oder starke organische Säuren, bevorzugt sind Salzsäure, oder Schwefelsäure. Das Lösungsmittel ist Wasser oder eine Mischung aus Wasser und einem unter den Reaktionsbedingungen inerten organischen Lösungsmittel. Die Reaktionstemperatur beträgt in der Regel zwischen -5°C und 50°C, vorzugsweise 10°C bis 20°C.

Die Umsetzung der erhaltenen Diazoniumsalze zur Iodverbindung (XII) erfolgt in der Regel ohne Isolierung und wird im gleichen wässrigen oder wässrig-organischen Lösungsmittel oder Lösungsmittelgemisch wie die Diazotierung durchgeführt. Bei der Umsetzung wird die Diazoniumgruppe durch das lodatom ausgetauscht, entweder durch das Anion des Diazoniumsalzes (wenn in der Säure X⁻ = I⁻) oder (falls X⁻ nicht I⁻ ist) durch die Reaktion mit zugefügtem lodid, z. B. Alkalimetalliodid, vorzugsweise Natriumiodid oder Kaliumiodid. Die Menge an lodid beträgt dabei beispielsweise 1.1 bis 1.5 Mol lodid pro Mol ursprünglich eingesetztem Anilin (XI). Die Reaktionstemperatur beläuft sich dabei im Allgemeinen auf 10°C bis 40°C, vorzugsweise 15°C bis 30°C (vgl. hierzu z.B. DE 19625831 und Bioorg. Med. Chem. 2004,12,2079) (Schema 3).

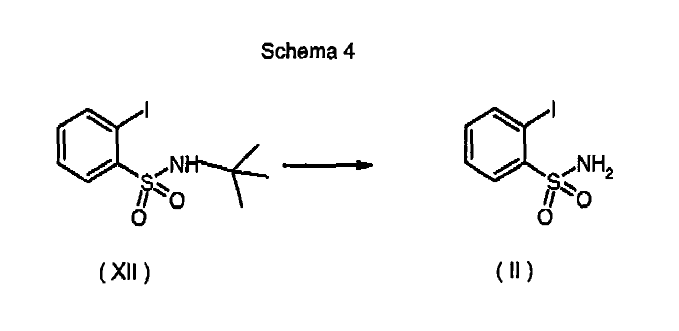

Die Abspaltung der tert.-Butyl-Schutzgruppe in (XII) zu 2-lodbenzolsulfonamid (II) erfolgt z.B. durch Behandlung mit einer starken Säure (siehe WO 89/10921). Als starke Säuren kommen z.B. Mineralsäuren, wie H₂SO₄ oder HCl, oder starke organische Säuren, wie Trifluoressigsäure in Frage. Die Reaktion erfolgt beispielsweise bei Temperaturen von -20°C bis zur jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden (Schema 4).

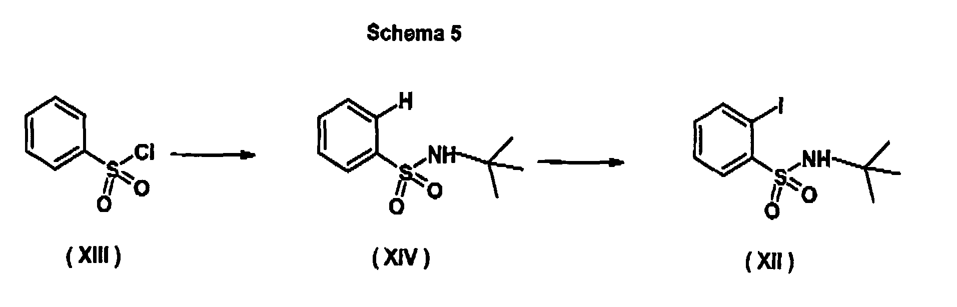

N-tert.-Butyl-2-iodbenzolsulfonsäureamid (XII) kann auch erhalten werden, indem man N-tert.-Butyl-benzolsuffonsäureamid (XIV), welches durch Umsetzung von kommerziell erhältlichem Benzolsulfonsäurechlorid (XIII) mit tert.-Butylamin (siehe Schema 1) erhalten werden kann, mit einer metallorganischen Verbindung, wie z.B. Alkyl- oder Aryllithium, vorzugsweise n- oder *sec*-Butyllithium in Hexan, gegebenfalls in Gegenwart eines (weiteren) inerten Verdünnungsmittels, wie z.B. Tetrahydrofuran, und unter Inertgasatmosphäre, wie z.B. unter Argon oder Stickstoff, bei Temperaturen zwischen -70°C und 20°C metalliert - d. h. das in (XIV) in ortho-Position zur SO₂NH-tert.-Sutyl-Gruppe befindliche Wasserstoffatom durch ein Metallatom ersetzt - und dann im gleichen Reaktionsmedium mit lod bei Temperaturen zwischen -100°C und 40°C, vorzugsweise zwischen -70°C und 20°C umsetzt, wodurch das Metallatom durch lod ersetzt (Schema 5) wird (siehe hierzu auch: V. Snieckus et al., J. Org. Chem. 2001, 66, 3662 und Synlett 2000, (9), 1294).

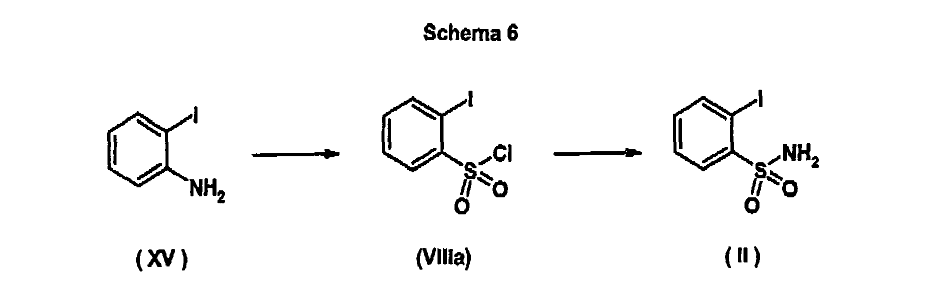

2-Iodbenzolsulfonamid (II) kann auch erhalten werden (Schema 6), indem man 2-lodbenzolsulfonsäurechlorid (VIIIa), welches durch Diazotierung der Aminogruppe in 2-lodanilin (XV) und nachfolgendem Austausch der resultierenden Diazogruppe durch eine Chlorsulfonylgruppe (wie in Schema 1 näher beschrieben) herstellt, mit Ammoniak umsetzt. Hierzu wird in inerte Lösungsmittel, vorzugsweise Dichlormethan oder Tetrahydrofuran, solange Ammoniakgas eingeleitet, bis kein Ammoniak mehr aufgenommen wird.
Alternativ kann 2-lodbenzolsulonsäurechlorid (VIIIa) auch durch Reaktion mit tert.-Butylamin (analog Schemata 1 und 5) in das N-tert-Butyl-2-lodbenzolsulfonamid (XII) überführt werden. Nachfolgend wird dann die tert.-Butyl-Schutzgruppe mitttels Säure entfernt (analog Schema 4) um 2-Iodbenzolsulfonamid (II) zu erhalten.

Die Suffonylcarbamate der allgemeinen Formel (IV) werden in Analogie zu an sich bekannten Reaktionen (vgl. EP-A-120 814) hergestellt. Man kann z.B. auch 2-Iodbenzolsulfonylisocyanat der Formel (VI) in glatter Reaktion in einem inerten Lösungsmittel, vorzugsweise Diethylether oder Dichlormethan mit Phenol in die Carbamate der Formel (IV) überführen, Die Aminoheterocyclen der Formel (V) sind bekannte, zum Teil im Handel erhältliche Synthesechemikalien.
Die Umsetzung der Sulfonylcarbamate der Formel (IV) mit den Aminoheterocyclen der Formel (V) erfolgt nach bekannten Verfahren (vgl. z.B. WO 2003 091228) (Schema 7).

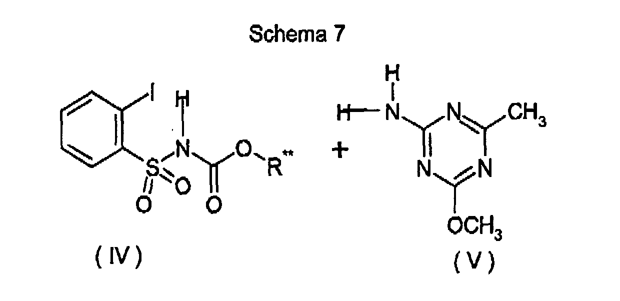

2-lodbenzolsulfanylisocyanat der Formel (VI) ist eine neue Verbindung und ist somit ebenfalls Gegenstand dieser Erfindung. Sie kann nach an sich bekannten Verfahren aus 2-Iodbenzolsulfonamid (II) hergestellt werden (vgl. DE 3208189, EP 23422, EP 64322, EP 44807, EP 216504). Man erhält das Suffonylisocyanat der Formel (VI), wenn man 2-lodbenzolsulfonamid (II) mit Phosgen, Diphosgen bzw. Thiophosgen, gegebenenfalls in Gegenwart eines Alkylisocyanats, wie z.B. Butylisocyanat, gegebenenfalls in Gegenwart eines Reaktionshilfsmittels, wie eines tertiären Amins, bevorzugt einem Diazabicyclo[2.2.2]octan, und in Gegenwart eines Verdünnungsmitels, wie Toluol, Xylol oder Chlorbenzol, bei Temperaturen zwischen 80°C und 150°C umsetzt und nach Ende der Umsetzung gegebenenfalls die flüchtigen Komponenten unter vermindertem Druck abdestilliert (Schema 8).

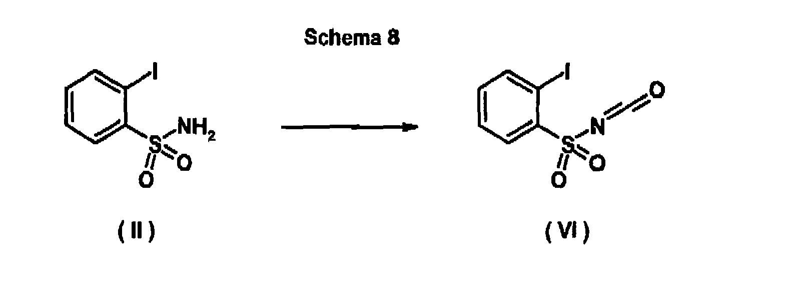

Die Umsetzung von 2-Iodbenzolsulfonylisocyanaten der Formel (VI) mit dem Aminotriazin der Formel (V) erfolgt z.B. nach bekannten Verfahren (vgl. WO 2003 091228) (Schema 9).

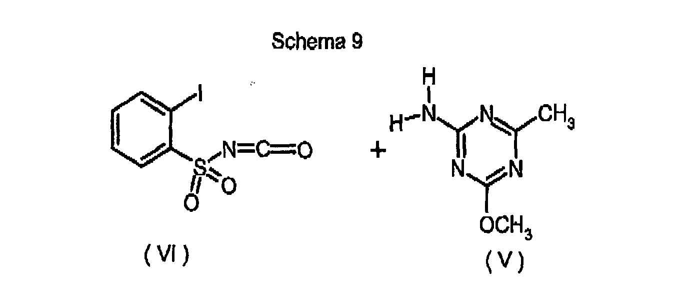

Die Isocyanate der allgemeinen Formel (VII) erhält man z.B. aus den Aminoheterocyclen des Typs (V) durch Behandlung mit Oxalylchlorid oder Phosphen (in Analogie nach Angew. Chem. 1971, 83, S. 407; EP 388 873). Die Umsetzung des lsocyanats des Typs (VII) mit 2-Iodbenzolsulfonamid (II) erfolgt z.B. in Analogie nach Variante c) (Schema 10).

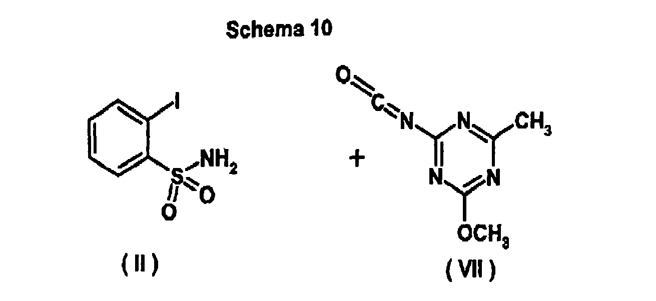

2-Iodbenzolsutfonsäurefluorid (VIIIb) kann auf verschiedene literaturbekannte Methoden hergestellt werden: i) aus 2-Iodbenzolsulfonamid (II) durch Diazotierung mit Alkalinitrit, beispielsweise Natriumnitrit, und nachfolgender Umsetzung mit Fluorwasserstoff (J. Am. Chem. Soc. 1951, 73, 1857); ii) Umsetzung von 2-lodbenzolsulfonsäurechlorid (VIIIa) mit Kaliumfluorid (J. Chem. Soc, Perkin Trans. 1, 1998, 5, 875); iii) Reaktion von 2-lodbenzolsulfonsäure (XVII) mit Fluorsulfonsäure (US 2686202).
2-lodbenzolsulfonsäurebromid (VIIIc) kann beispielsweise durch Umsetzung von 2-lodbenzolsulfonsäurechlorid (VIIIa) mit Bromwasserstoff in Essigsäure synthetisiert werden (Dokl. Akad. Nauk SSR 1955,103, 627).

In einer Ausführungsform der Variante f) wird die durch Umsetzung des Sulfonsäurehalogenids (VIII) mit einem Cyanat erhaltene Reaktionsmischung direkt für die Kupplung mit dem Aminotriazin der Formel (V) zur Synthese der Vorstufe (Neutralverbindung) der Formel (I) eingesetzt (vgl. hierzu WO 2003 091228 und US 5550238).

Die erfindungsgemäßen Salze, insbesondere solche der Formel (I) lassen sich aus der Neutralform des Sulfonylhamstoffs oder Sulfonythamstoffmetallsalzen, insbesondere Alkalimetallsalzen (siehe z.B. EP-A-30138, EP-A-7687) oder auch ausgehend von Sulfonamidsalzen z.B. auf folgende Weise herstellen:
1. Deprotonierung des neutralen Sulfonylhemstoffs (XX) mit einer geeigneten Base der Formel M⁺B⁻ (Schema 11), wobei B⁻ zum Beispiel Hydrid, Hydroxy- oder Alkoxyanionen, wie Methoxy, Ethoxy, n-Propoxy, i-Propoxy, n-Butoxy oder t-Butoxy darstellen. 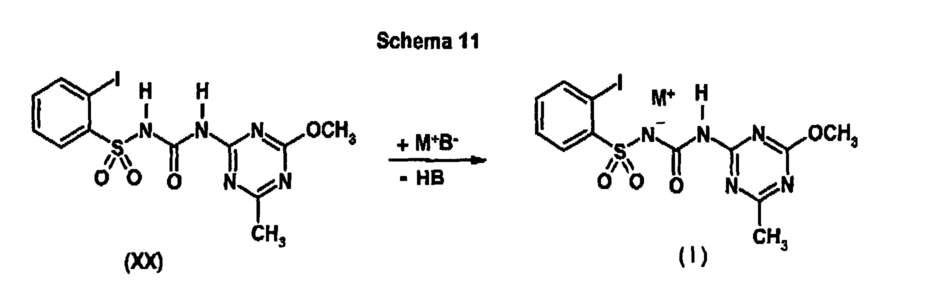 Hierzu wird der Suffonylhamstoff der Formel (I) in einem inerten Lösungsmittel oder Lösungsmittelgemisch gelöst oder suspendiert und mit einem Äquivalent an M⁺B⁻ bei Temperaturen zwischen -20°C uns 100°C, vorzugsweise zwischen -10°C und 50°C umgesetzt.
2. Umsalzung von Metallsalzen des Suffonylhamstoffs der Formel (la), worin Met⁺ ein Metallkation, bevorzugt ein Alkalimetallion wie Na⁺ oder K⁺ ist, mit geeigneten Reagentien der Formel M⁺X⁻ (Schema 12), wobei M⁺ ein Ammoniumion ist und X⁻ ein Anion, beispielsweise ein Halogenanion wie F⁻, Cl⁻ oder Br⁻ bedeutet oder ein Phosphat-, Sulfat- oder Carboxylatanion sein kann, wobei diese Definition anorganische sowie organische Salze einschließt, wie sie z. B. in der Tensidchemie gebräuchlich sind (z. B. organische Phosphatahionen, Phosphonatanionen, Sulfatanionen, Sulfonatanionen, Carboxylate). 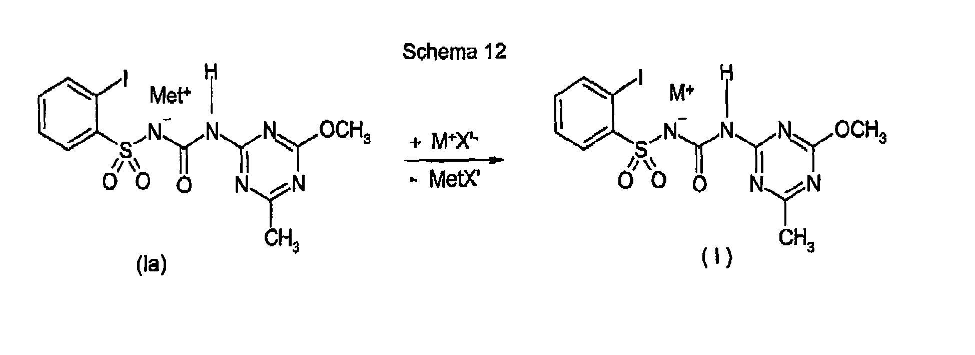 Hierzu werden die erfindungsgemäßen Metallsalze, z. B. Alkalisalze (z.B. Met⁺ = Na⁺, K⁺) des Sulfonylhamstoffs in einem inerten Lösungsmittel oder Lösungsmittelgemisch gelöst und mit einem Äquivalent der Reagenz M⁺X⁻umgesetzt. Nach beendeter Reaktion kann durch Filtration das als Nebenprodukt anfallende Salz, z. B. Alkalisalz (wie NaCl) abgetrennt werden.
3. In-situ Deprotonierung und Umsalzung (Schema 13), ausgehend vom neutalen Sulfonylhamstoff (XX) mit geeigneten Reagentien a) der Formel M⁺X⁻, wobei M⁺ ein Ammoniumion ist und X⁻ ein Anion beispielsweise ein Halogenanion wie F⁻, Cl⁻ oder Br⁻ bedeutet oder ein Phosphat-, Sulfat- oder Carboxylatanion sein kann, wobei diese Definition anorganische sowie organische Salze einschließt, wie sie z. B, in der Tensidchemie gebräuchlich sind (z. B. organische Phosphatanionen, Phosphonatanionen, Suffatanionen, Sulfonatanionen, Carboxylate) und b) der Formel Met⁺ B', worin Met⁺ ein Metallkation, insbesondere ein Alkalimetallkation wie Na⁺ oder K⁺ ist und B⁻ eine geeignete Base, z. B. ein Hydroxy- oder Alkoxyanion wie Methoxy, Ethoxy, *n*-Propoxy, *i*-Propoxy oder n-Butoxy oder das Anion eines alkoxylierten, z. B. ethoxylierten oder propoxylierten, (C₈-C₄₀)-Alkoheis ist. 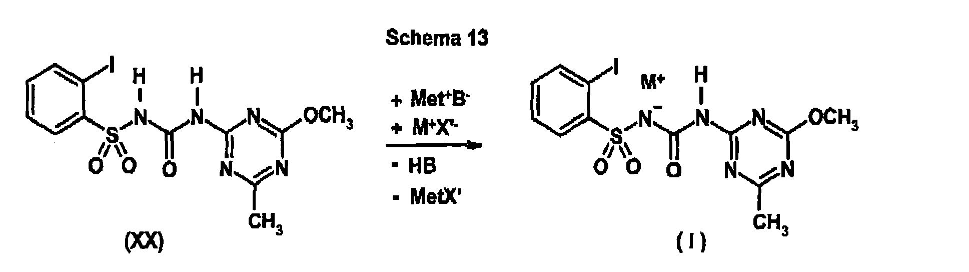 Hierzu wird der neutrale Sulfonalharnstoff (XX) in einem inerten Lösungsmittel oder Lösungsmittelgemisch gelöst und mit je einem Äquivalent der Reagentien M⁺C⁻ und MetB umgesetzt. Nach beendeter Reaktion kann durch Filtration das als Nebenprodukt anfallende Metallsalz, insbesondere Alkalisalz (z. B. NaCl) abgetrennt werden.
4. Umsetzungen des neutralen Sulfonylhamstoffs (XX) mit geeigneten Zwitterionen, z. B. (Schema 14) 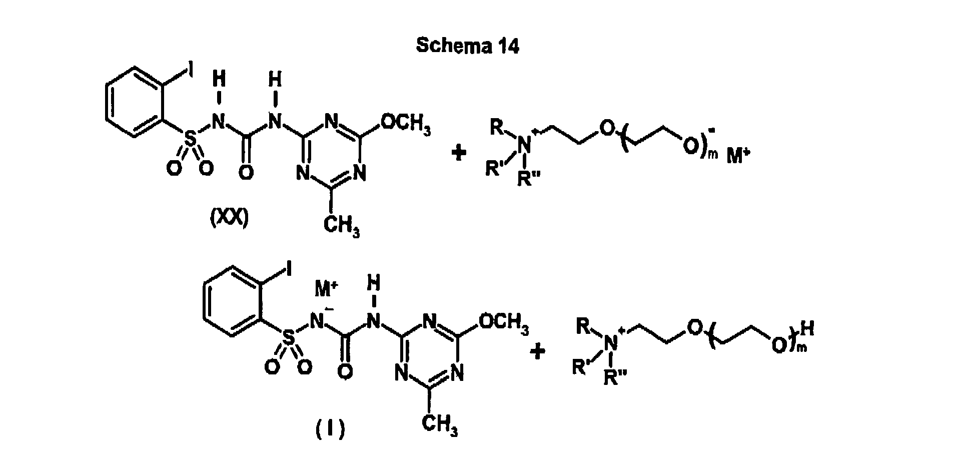 wobei R ein (C₁-C₂₀)-Kohlenstoffrest wie (C₁-C₁₀)-Alkyl ist, R' und R" gleich oder voneinander verschieden sind und Wasserstoff oder (C₁-C₃₀)-Kohlenwasserstoffreste wie (C₁-C₁₀)-Alkyl sind und m eine ganze Zahl von 0 bis 100 ist. Hierzu wird der neutrale Sulfonylhamstoff (XX) mit einem Zwitterion wie z. B. im Schema 14 angegeben in einem inerten Lösungsmittel z. B. Methanol, Tetrahydrofuran oder Methylenchlorid oder Lösungsmittelgemisch bei Temperaturen zwischen -20°C und 100°C, vorzugsweise -10°C und 80°C in äquimolaren Verhältnissen umgesetzt.
5. Umsetzung eines Sulfonsäureamidsalzes der Formel (IIa) mit dem Isocyanat (VII) (Schema 15). 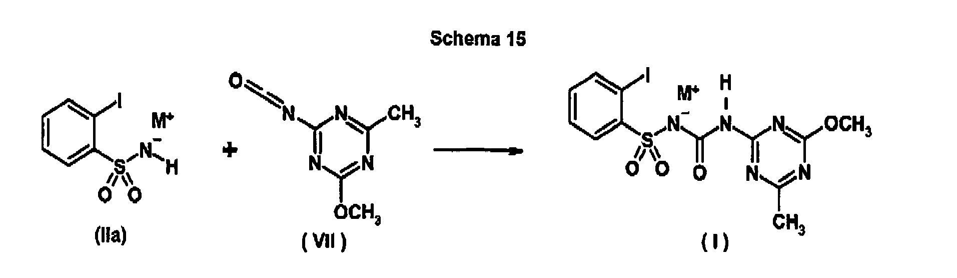 Die Umsetzung erfolgt in einem inerten Lösungsmittel oder Lösungsmittelgemisch - wie z. B. Tetrahydrofuran - bei Temperaturen zwischen -20°C und 100°C, vorzugsweise zwischen -10°C und 70°C, indem man das Isocyanat (VII) äquimolar mit dem Sulfonsäureamidsalz der Formel (IIa) umsetzt. Dabei kann das Sulfonsäureamidsalz der Formel (IIa) direkt eingesetzt werden oder in-situ gebildet werden - z. B. durch Reaktion des entsprechenden Sulfonsäureamids der Formel (II) mit einer geeigneten Base M⁺X⁻, worin M⁺ ein Ammoniumion ist und X⁻ z. B. ein Hydroxy- oder Alkoxyanion.
6. Umsetzung eines Sulfonsäureamidsalzes der Formel (IIa) mit einem Carbamat der allgemeinen Formel (III) (Schema 16) 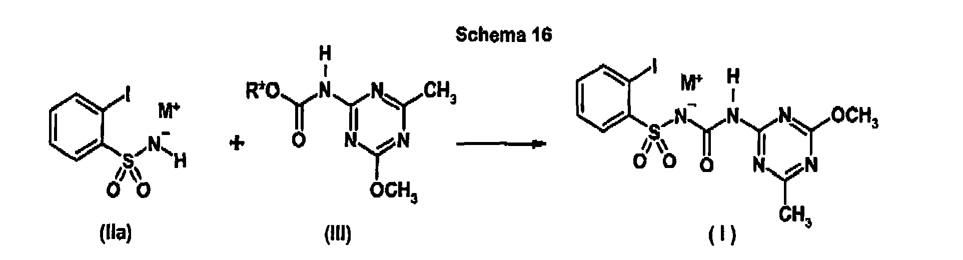 Die Umsetzung erfolgt in einem inerten Lösungsmittel (oder Lösungsmittelgemisch) - wie z.B. Tetrahydrofuran - bei Temperaturen zwischen -20°C und 100°C, vorzugsweise zwischen -10°C und 70°C, indem man das Carbamat der Formel (III) äquimolar mit mit dem Sulfonsäureamidsalz der Formel (IIa) umsetzt. Dabei kann das Suffonsäureamidsalz der Formel (IIa) direkt eingesetzt oder in-situ gebildet werden - z. B. durch Reaktion des entsprechenden Sulfonsäureamids mit einer geeigneten Base M⁺X⁻, worin M⁺ ein Ammoniumion ist und X⁻ z. B. ein Hydroxy- oder Alkoxyanion.
7. Umsetzung des neutralen Sulfonylhamstoffs (XX) mit einem primären, sekundären oder tertiären Amin NRR'R" (Schema 17), worin R, R' und R" einem gleichen oder verschiedenenen Rest ausgwählt aus der Gruppe bestehend aus:
   (a) H,
   (b) einem Ammonium-Ion, bei dem gegebenenfalls ein, zwei, drei oder alle vier Wasserstoffatome, durch gleiche oder verschiedene Reste aus der Gruppe (C₁-C₄)-Alkyl, Hydroxy-(C₁-C₄)-Alkyl, (C₃-C₆)-Cycloalkyl, (C₁-C₄)-Alkoxy-(C₁-C₄)-alkyl, Hydroxy-(C₁-C₄)-alkoxy-(C₁-C₄)-alkyl, (C₁-C₆)-Mercaptoalkyl, Phenyl oder Benzyl substituiert sind, wobei die zuvor genannten Reste gegebenenfalls durch einen oder mehrere, gleiche oder verschiedene Reste aus der Gruppe Halogen, wie F, Cl, Br oder I, Nitro, Cyano, Azido, (C₁-C₆)-Alkyl, (C₁-C₆)-Haloalkyl, (C₃-C₆)-Cycloalkyl, (C₁-C₆)Alkoxy, (C₁-C₆)-Haloalkoxy und Phenyl substituiert sind, und wobei jeweils zwei Substituenten am N-Atom zusammen gegebenfalls einen unsubstituierten gegebenenfalls einem substituierten Ring bilden, oder
   (c) einer gegebenenfalls einfach oder mehrfach annellierten und/oder durch (C₁-C₄)-Alkyl substituierten gesättigten oder ungesättigten/aromatischen N-haltigen heterocyclischen ionischen Verbindung mit 1-10 C-Atomen im Ringsystem
   entsprechen

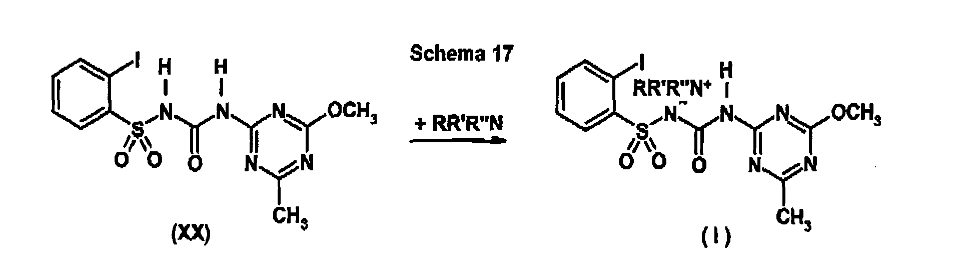

Diese Umsetzung erfolgt zwischen Temperaturen von -20°C bis 100°C, bevorzugt zwischen -10°C und 50°C, in inerten Lösungsmittel, wie z. B. Tetrahydrofuran, Methylenchlorid oder Methanol oder Gemischen aus Lösungsmitteln, statt.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Kollektionen aus erfindungsgemäßen Salzen, insbesondere solchen der Formel (I), die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren, Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Für die Durchführung mikrowellenunterstützter Synthesen kann ein Mikrowellengerät, z.B. Modell "Discover" der Firma CEM GmbH Mikrowellen-Analysentechnik, Carl-Friedrich-Gauß-Str, 9, 47475 Kamp-Lintfort verwendet werden.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, England, H+P Labortechnik GmbH, Bruckmannring 28, 85764 Oberschleißheim, Deutschland oder der Firma Radleys, Shirehill, Saffron Walden, Essex, CB 11 3AZ, England angeboten werden. Für die parallelisierte Aufreinigung von Verbindungen der allgemeinen Formel (I) beziehungsweise von bei der Herstellung anfallende Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen Methoden kann die Herstellung der erfindungsgemäßen Salze, insbesondere von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Cambinatorial Index", Verlag Academic Press, 1998.

Die Verwendung von festphasenunterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert die erfindungsgemäßen Salze, insbesondere Verbindungen der Formel (I), in Form von Substanzkollektionen, die Bibliotheken genannt werden. Gegenstand der vorliegenden Erfindung sind auch Bibliotheken, die mindestens zwei erfindungsgemäße Verbindungen, insbesondere Verbindungen der Formel (I) enthalten.

Die erfindungsgemäßen Salze, insbesondere Verbindungen der Formel (I), weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.
Werden die erfindungsgemäßen Salze, insbesondere Verbindungen der Formel (I), vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt erreichten Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Salze, insbesondere die Verbindungen der Formel (I), eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Hafer, Reis, Mais, Zuckerrohr und Plantagenkulturen nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Weiterhin weisen die erfindungsgemäßen Salze, insbesondere Verbindungen der Formel (I) sehr vorteilhafte Eigenschaften hinsichtlich des Verhaltens in der Umwelt, insbesondere bzgl. des Nachbauverhaltens auf, d.h. gegenüber den erfindungsgemäßen Verbindungen der Formel (I) ansonsten empfindlichen Kulturen, wie beispielsweise Zuckerrübe, Sonnenblume oder Cruciferen, wie beispielsweise Raps, Senf und Rübsen.

Darüberhinaus weisen die erfindungsgemäßen Salze, insbesondere die Verbindungen der Formel (I), hervorragende wachstumsregulatorische Eigenschaften bei Kulturpflanzen auf. Sie greifen regulierend in den pflanzeneigenen Stoffwechsel ein und können damit zur gezielten Beeinflussung von Ptlanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregutatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmter Herbizide, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller lnhaltsstoffe. So sind transgene Pflanzen mit erhöhtem Stärkegehalt oder veränderter Qualität der Stärke oder solche mit anderer Fettsäurezusammensetzung des Emteguts bekannt. Gleichermaßen können die Wirkstoffe aufgrund ihrer herbiziden und pflanzenwachstumsregulatorischen Eigenschaften auch zur Bekämpfung von Schadpflanzen in Kulturen von bekannten oder noch zu entwickelnden durch Mutatantenselektion erhaltenen Pflanzen eingesetzt werden.

Bevorzugt ist die Anwendung der erfindungsgemäßen Salze, insbesondere von Verbindungen der Formel (I), in wirtschaftlich bedeutenden transgenen oder durch Mutantenselektion erhaltenen Kulturen von Nutz- und Zierpflanzen, z. B. von Getreide wie Weizen, Gerste, Roggen, Hafer, Hirse, Reis, Maniok und Mais oder auch Kulturen von Raps, Kartoffel, Tomate, Erbse und anderen Gemüsesorten.

Vorzugsweise können die erfindungsgemäßen Salze, insbesondere die Verbindungen der Formel (I), als Herbizide in Nutzpflanzenkulturen eingesetzt werden, welche gegenüber den phytotoxischen Wirkungen der Herbizide resistent sind bzw. gentechnisch resistent gemacht worden sind, oder durch Mutantenselektion erhalten wurden. Ebenso bevorzugt können die erfindungsgemäßen Salze, insbesondere die Verbindungen der Formel (I), als Herbizide in Nutzpflanzenkulturen eingesetzt werden, die ein Kreuzungsprodukt aus gentechnisch resistent gemachten Pflanzen und durch Mutantenselektion erhaltenen Pflanzen darstellen, wie z.B. in WO 2007/024782 beschrieben.

Allgemein bekannte Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten.

Ebenso können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1(1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind. Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Salze, insbesondere Verbindungen der Formel (I) in transgenen oder durch Mutantenselektion erhaltenen Kulturen oder Kreuzungen/Hybriden derselben eingesetzt werden, welche gegen Herbizide aus der Gruppe der Suffonylhamstoffe, Glufosinate-ammonium oder Glyphosateisopropylammonium und analoge Wirkstoffe resistent sind.

Bei der Anwendung der erfindungsgemäßen Wirkstoffe in transgenen oder durch Mutantenselektion erhaltenen Kulturen oder Kreuzungen derselben treten neben den in anderen Kulturen zu beobachtenden Wirkungen gegenüber Schadpflanzen oftmals Wirkungen auf, die für die Applikation in der jeweiligen transgenen oder durch Mutantenselektion erhaltenen Kulturen oder Kreuzungen derselben spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.
Gegenstand der Erfindung ist deshalb auch die Verwendung der erfindungsgemäßen Salzen, insbesondere von Verbindungen der Formel (I), als Herbizide zur Bekämpfung von Schadpflanzen in transgenen oder durch Mutantenselektion erhaltenen Kulturpflanzen oder Kreuzungen derselben.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die die Verbindungen der Formel (I) enthalten.
Die erfindungsgemäßen Salze, insbesondere die Verbindungen der Formel (I), können auf verschiedene Arten formuliert werden, je nachdem welche biologischen und/oder chemisch-physikalischen Parameter vorgegeben sind. Als Formullerungsmöglichkoften kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Fonnutierungen. Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd, London.
Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H,v. Olphen, "Introduction to Clay Colloid Chemistry"; 2nd Ed., J. Wiley & Sons, N.Y.; C. Marsden, "Solvents Gulde"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.
Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.
Spritzpulver sind in Wasser gleichmäßig dispergierbare Präparate, die neben dem Wirkstoff außer einem Verdünnungs- oder lnertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthyimethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formullerungshilfsmitteln vermischt.
Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenaxid-Ethylenaxid-Kandensationsprodukte, Alkylpolyether, Sorbitan-ester wie z.B. Sorbitanfettsäureester oder Polyoxethylensorbitanester wie z.B. Polyoxyethylensorbitanfettsäureester.
Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Nass-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemittein, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1987, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.
Die agrochemischen Zubereitungen enthalten in der Regel 0,1 bis 99 Gew.-%, vorzugsweise 0,1 bis 95 Gew.%, insbesondere bevorzugt 0.5 bis 90 Gew.-% Wirkstoff der erfindungsgemäßen Salze, insbesondere der Verbindungen der Formel (I).

In Spritzpulvern beträgt die Wirkstoffkonzentration z.B. etwa 10 bis 90 Gew.-%, der Rest zu 100 Gew.-% besteht aus üblichen Formulierungsbestandtellen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.
Daneben enthalten die genannten Wirkstoffformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, kömige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Die herbizide Wirkung der erfindungsgemäßen Herbizid-Kombinationen kann z.B. auch durch oberflächenaktive Substanzen verbessert werden, vorzugsweise durch Netzmittel aus der Reihe der Fettalkohol-Polyglykolether. Die Fettalkohol-Polyglykolether enthalten vorzugsweise 10 - 18 C-Atome im Fettalkoholrest und 2 - 20 Ethylenoxideinheiten im Polyglykoletherteil. Die Fettalkohol-Polyglykolether können nichtionisch vorliegen, oder ionisch, z.B. in Form von-Fettalkohol-Polyglykolethersulfaten, vorliegen, die z.B. als Alkalisalze (z.B. Natrium- und Kaliumsalze) oder Ammoniumsalze, oder auch als Erdalkalisalze wie Magnesiumsalze verwendet werden, wie C₁₂/C₁₄-Fettalkohol-diglykolethersulfat-Natrium (Genapol^{®} LRO, Clariant GmbH); siehe z.B. EP-A-0476555, EP-A-0048436, EP-A-0336151 oder US-A-4,400,196 sowie Proc. EWRS Symp. "Factors Affecting Herbicidal Activity and Selectivity", 227 - 232 (1988). Nichtionische Fettalkohol-Polyglykolether sind beispielsweise 2 - 20, vorzugsweise 3 - 15, Ethylenoxideinheiten enthaltende (C₁₀- C₁₈)-, vorzugsweise (C₁₀-C₁₄)-Fettalkohol-Polyglykolether (z.B. Isotridecylalkohol-Polyglykolether) z.B. aus der Genapol^{®} X-Reihe wie Genapol^{®} X-030, Genapol^{®} X-060, Genapol^{®} X-080 oder Genapol^{®} X-150 (alle von Clariant GmbH).

Die vorliegende Erfindung umfaßt ferner die Kombination von Komponenten A und B mit den vorgängig genannten Netzmitteln aus der Reihe der Fettalkohol-Polyglykolether, die vorzugsweise 10 - 18 C-Atome im Fettalkoholrest und 2 - 20 Ethylenoxideinheiten im Polyglykoletherteil enthalten und nichtionisch oder ionisch (z.B. als Fettalkohol-polyglykolethersulfate) vorliegen können. Bevorzugt sind C₁₂/C₁₄-Fettalkohol-diglykolethersulfat-Natrium (Genapol^{®} LRO, Clariant GmbH) und Isotridecylalkohol-Polyglykolether, mit 3 - 15 Ethylenoxideneinheiten, z.B. aus der Genapol^{®} X-Reihe wie Genapol^{®} X-030, Genapol^{®} X-060, Genapol^{®} X-080 und Genapol^{®} X-150 (alle von Clariant GmbH). Weiterhin ist bekannt, daß Fettalkohol-Polyglykolether wie nichtionische oder ionische Fettalkohol-polyglykolether (z.B. Fettalkohol-Polyglykolethersulfate) auch als Penetrationshilfsmittel und Wirkungsverstärker für eine Reihe anderer Herbizide, unter anderem auch für Herbizide aus der Reihe der Imidazolinone geeignet sind (siehe z.B. EP-A-0502014).

Weiterhin ist bekannt, daß Fettalkohol-Polyglykolether wie nichtionische oder ionische Fettalkohol-Polyglykolether (z.B. Fettalkohol-Polyglykolethersulfate) auch als Penetrationshilfsmittel und Wirkungsverstärker für eine Reihe anderer Herbizide, unter anderem auch für Herbizide aus der Reihe der Imidazolinone geeignet sind (siehe z.B. EP-A-0502014).

Die herbizide Wirkung der erfindungsgemäßen Herbizid-Kombinationen kann auch durch die Verwendung von Pflanzenölen verstärkt werden. Unter dem Begriff Pflanzenöle werden Öle aus ölliefemden Pflanzenarten wie Sojaöl, Rapsöl, Maiskeimöl, Sonnenblumenöl, Baumwollsaatöl, Leinöl, Kokosöl, Palmöl, Distelöl oder Rhizinusöl, insbesondere Rapsöl verstanden, sowie deren Umesterungsprodukte, z.B. Alkylester wie Rapsölmethylester oder Rapsölethylester.

Die Pflanzenöle sind bevorzugt Ester von C₁₀-C₂₂-, vorzugsweise C₁₂-C₂₀-Fettsäuren. Die C₁₀-C₂₂-Fettsäureester sind beispielsweise Ester ungesättigter oder gesättigter C₁₀-C₂₂-Fettsäuren, insbesondere mit gerader Kohlenstoffatomzahl, z.B. Erucasäure, Laurinsäure, Palmitinsäure und insbesondere C₁₈-Fettsäuren wie Stearinsäure, Ölsäure, Linolsäure oder Linolensäure.

Beispiele für C₁₀-C₂₂-Fettsäure-Ester sind Ester, die durch Umsetzung von Glycerin oder Glykol mit den C₁₀-C₂₂-Fettsäuren erhalten werden, wie sie z.B. in Ölen aus ölliefernden Pflanzenarten enthalten sind, oder C₁-C₂₀-Alkyl-C₁₀C₂₂-Fettsäure-Ester, wie sie z.B. durch Umesterung der vorgenannten Glycerin- oder Glykol-C₁₀-C₂₂-Fettsäure-Ester mit C₁-C₂₀-Alkoholen (z.B. Methanol, Ethanol, Propanol oder Butanol) erhalten werden können. Die Umesterung kann nach bekannten Methoden erfolgen, wie sie z.B. beschrieben sind im Römpp Chemie Lexikon, 9. Auflage, Band 2, Seite 1343, Thieme Verlag Stuttgart.

Als C₁-C₂₀-Alkyl-C₁₀-C₂₂-Fettsäure-Ester bevorzugt sind Methylester, Ethylester, Propylester, Butylester, 2-ethyl-hexylester und Dodecylester. Als Glykol- und Glycerin-C₁₀-C₂₂-Fettsäure-Ester bevorzugt sind die einheitlichen oder gemischten Glykolester und Glycerinester von C₁₀-C₂₂-Fettsäuren, insbesondere solcher Fettsäuren mit gerader Anzahl an Kohlenstoffatomen, z.B. Erucasäure, Laurinsäure, Palmitinsäure und insbesondere C₁₈-Fettsäuren wie Stearinsäure, Ölsäure, Linolsäure oder Linolensäure.

Die Pflanzenöle können in den erfindungsgemäßen herbiziden Mitteln z.B. in Form kommerziell erhältlicher ölhaltiger Formulierungszusatzstoffe, insbesondere solcher auf Basis von Rapsöl wie Hasten^{®} (Victorian Chemical Company, Australien, nachfolgend Hasten genannt, Hauptbestandteil: Rapsölethylester), Actirob^{®}B (Novance, Frankreich, nachfolgend ActirobB genannt, Hauptbestandteil: Rapsölmethylester), Rako-Binol^{®} (Bayer AG, Deutschland, nachfolgend Rako-Binol genannt, Hauptbestandteil: Rapsöl), Renol^{®} (Stefes, Deutschland, nachfolgend Renol genannt, Pflanzenölbestandteil: Rapsölmethylester) oder Stefes Mero^{®} (Stefes, Deutschland, nachfolgend Mero genannt, Hauptbestandteil: Rapsölmethylester) enthalten sein.

Die vorliegende Erfindung umfasst in einer weiteren Ausführungsform Kombinationen mit den vorgängig genannten Pflanzenölen wie Rapsöl, bevorzugt in Form kommerziell erhältlicher ölhaltiger Formulierungszusatzstoffe, insbesondere solcher auf Basis von Rapsöl wie Hasten^{®} (Victorian Chemical Company, Australien, nachfolgend Hasten genannt, Hauptbestandteil: Rapsölethylester), Actirob^{®}B (Novance, Frankreich, nachfolgend ActirobB genannt, Hauptbestandteil; Rapsölmethylester), Rako-Binol^{®} (Bayer AG, Deutschland, nachfolgend Rako-Binol genannt, Hauptbestandteil: Rapsöl), Renol^{®} (Stefes, Deutschland, nachfolgend Renol genannt, Pflanzenölbestandteil: Rapsölmethylester) oder Stefes Mero^{®} (Stefes, Deutschland, nachfolgend Mero genannt, Hauptbestandteil: Rapsölmethylester).

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die erfindungsgemäßen Salze, insbesondere die Verbindungen der Formel (I), können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Salze, insbesondere für die Verbindungen der Formel (I) in Mischungsformulierungen oder im Tank-Mix sind beispielsweise bekannte, vorzugsweise herbizide Wirkstoffe, die auf einer Inhibition von beispielsweise Acetolactat-Synthase, Acetyl-Coenzym-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder in dem Handbuch "The Pesticide Manual", 12. Auflage 2000, oder 13. Auflage 2003 oder 14. Auflage 2006/2007, oder in dem entsprechenden "e-Pesticide Manual", Version 4 (2006), jeweils herausgegeben vom British Crop Protection Council, (im Folgenden auch kurz "PM"), und dort zitierter Literatur beschrieben. Listen von "Common names" sind auch in "The Compendium of Pesticide Common Names" im Internet verfügbar. Als literaturbekannte Herbizide, die mit den Verbindungen der Formel (I) kombiniert werden können, sind z.B. die Wirkstoffe aus der nachfolgenden Tabelle 1 zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor, acibenzolar-S-methyl; acifluorfen(-sodium); aclonifen; AD-67; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester, alachlor; alloxydim(-sodium); ametryn; amicarbazone, amidochlor, amidosulfuron; aminopyralid; amitrol; ammonium pelargonate; AMS, d.h. ammonium sulfamat; ancimidol; anilofos; asulam; atrazine; aviglycine; azafenidin, azimsulfuron (DPX-A8947); aziprotryn; barban; BAS 516 H, d.h. 5-Fluor-2-phenyl-4H-3,1-benzoxazin-4-on; beflubutamid (UBH-509), benazolin(-ethyl); bencarbazone; benfluralin; benfuresate; bensulfuron(-methyl); bensulide; bentazone; benzfendizone; benzobicyclon, benzofenap; benzofluor; benzoylprop(-ethyl); benzthiazuron; bilanaphos; bifenox; bispyribac(-sodium) (KIH-2023); borax; bromacil; bromobutide; bromofenoxim; bromoxynil; bromuron; buminafos; busoxinone; butachlor; butafenacil; butamifos; butenachlor (KH-218); buthidazole; butralin; butroxydim; butylate; cafenstrole (CH-900); caloxydim; carbetamide; carfentrazone(-ethyl); catechin; CDAA, d.h. 2-Chlor-N,N-di-2-propenylacetamid; CDEC, d.h. Diethyldithiocarbaminsäure-2,chlorallylester; chlormesulon; chlomethoxyfen; chloramben; chlorazifop-butyl; chlorbromuron; chlorbufam; chlorfenac; chlorfenprop; chlorflurecol(-methyl); chlorflurenol(-methyl); chloridazon; chlorimuron(-ethyl); chlormequat(-chloride); chlomitrofen; chlorophthalim (MK-616); chlorotoluron; chloroxuron; chlorpropham; chlorsulfuron; chlorthal-dimethyl; chlorthiamid; chlortoluron; cinidon(-methyl und -ethyl); cinmethylin; cinosulfuron; clefoxydim; clethodim; clodinafop und dessen Esterderivate (z.B. clodinafop-propargyl); clofencet; clomazone; clomeprop; cloprop; cloproxydim; clopyralid; clopyrasulfuron(-methyl); cloransulam(-methyl); cumyluron (JC 940); cyanamide; cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-D; 2,4-DB; dalapon; daminozide; dazomet; n-decanol; desmedipham; desmetryn; di-allate; dicamba; dichlobenil; dichlormid; dichlorprop(-P)-salze; diclofop und dessen Ester wie diclofop-methyl; diclofop-P(-methyl); diclosulam; diethatyl(-ethyl); difenoxuron; difenzoquat(-metilsulfate); diflufenican; diflufenzopyr(-sodium); dimefuron; dimepiperate; dimethachlor; dimethametryn; dimethazone; dimethenamid (SAN-582H), dimethenamide-P; dimethylarsinic acid; dimethipin; dimetrasulfuron; dimexyflam; dinitramine; dinoseb; dinoterb; diphenamid; dipropetryn; diquat-salze; dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan; EPTC; esprocarb; efhatfluralin; ethametsulfuron-methyl; ethephon; ethidimuron; ethiozin; ethofumesate; ethoxyfen und dessen Ester (z.B. Ethylester, HN-252); ethoxysulfuron; etobenzanid (HW 52); F5231, d.h. N-[2-Chlor-4-fluor-5-[4-(3-fluorpmpyl)-4,5-dihydro-5-oxo-1H-tetrazol-1-yl]-phenyl]-ethansulfonamid; fenchlorazole(-ethyl); fenclorim; fenoprop; fenoxan, fenoxaprop und fenoxaprop-P sowie deren Ester, z.B. fenoxaprop-P-ethyl und fenoxaprop-ethyl; fenoxydim; fentrazamide; fenuron; ferrous sulfate; flamprop(-methyl oder-isopropyl oder -isopropyl-L); flamprop-M(-methyl oder-isopropyl); flazasulfuron; floazulate (JV-485); florasulam; fluazifop und fluazifop-P und deren Ester, z.B. fluazifop-butyl und fluazifop-P-butyl; fluazolate; flucarbazone(-sodium); flucetosulfuron; fluchloralin; flufenacet; flufenpyr(-ethyl); flumetralin; flumetsulam; flumeturon; flumiclorac(-pentyl); flumioxazin (S-482); flumipropyn; fluometuron; fluorochloridone; fluorodifen; fluoroglycofen(-ethyl); flupoxam (KNW-739); flupropacil (UBIC-4243); flupropanoate; flupyrsulfuron(-methyl)(-sodium); flurenol(-butyl); fluridone; flurochloridone; fluroxypyr(-meptyl); flurprimidol; flurtamone; fluthiacet(-methyl) (KIH-9201); fluthiamide; fluxofenim; fomesafen; foramsulfuron; forchlorfenuron; fosamine; furyloxyfen; gibberillic acid; glufosinate(-ammonium); glyphosate(-isopropylammonium); halosafen; halosulfuron(-methyl); haloxyfop und dessen Ester; haloxyfop-P (= R-haloxyfop) und dessen Ester, HC-252; hexazinone; HNPC-C9908, d.h. 2-[[[([4-Methoxy-6-(methylthio)-2-pyrimidinyl]amino]carbonyl]amino]sulfonyl]-benzoesäuremethylester; imazamethabenz(-methyl); imazamox; imazapic; imazapyr, imazaquin und Salze wie das Ammoniumsalz; imazethapyr, imazosulfuron; inabenfide; indanofan; iodosulfuron-methyl(-sodium); ioxynil; isocarbamid; isopropalin; isoproturon; isouron; isoxaben; isoxachlortole; isoxaflutole; isoxapyrifop; karbutilate; lactofen; lenacil; linuron; Maleinsäurehydrazid (MH); MBTA; MCPA; MCPB; mecoprop(-P); mefenacet; mefluidide; mepiquat(-chloride); mesosulfuron(-methyl); mesotrione; metam; metamifop; metamitron; metazachlor; methabenzthiazuron; metham; methazole; methoxyphenone; methylarsonic acid; methyl-cyclopropene; methyldymron; methylisothiocyanate; methabenzthiazuron; metobenzuron; metobromuron; (alpha-)metolachlor; metosulam (XRD 511); metoxuron; metribuzin; metsulfuron-methyl; molinate; monalide; monocarbamide dihydrogensulfate; monolinuron; monuron; monosulfuron; MT 128, d.h. 6-Chlor-N-(3-chlor2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclofen; nitralin; nitrofen; nitrophenolate mixture; nitrofluorfen; nonanoic acid; norflurazon; orbencarb; orthosulfamuron; oxabetrinil; oryzalin; oxadiargyl (RP-020630); oxadiazon; oxasulfuron; oxaziclomefone; oxyfluorfen; paciobutrazol; paraquat(-dichloride); pebulate; pelargonic acid; pendimethalin; penoxulam; pentachlorophenol; pentanochlor; pentoxazone; perfluidone; pethoxamid; phenisopham; phenmedipham; picloram; picolinafen; pinoxaden; piperophos; piributicarb; pirifenop-butyl; pretilachlor; primisulfuron(-methyl); probenazole; procarbazone-(sodium); procyazine; prodiamine; profluralin; profoxydim; prohexadione(-calcium); prohydrojasmon; proglinazine(-ethyl); prometon; prometryn; propachlor; propanil; propaquizafop und dessen Ester; propazine; propham; propisochlor; propoxycarbazone(-sodium) (MKH-6561); propyzamide; prosulfalin; prosulfocarb; prosulfuron (CGA-152005); prynachlor; pyraclonil; pyraflufen(-ethyl) (ET-751); pyrasulfotole; pyrazolynate; pyrazon; pyrazosulfuron(-ethyl); pyrazoxyfen; pyribambenz-isopropyl (ZJ 0702); pyrimbambenz-propyl (ZJ 0273); pyribenzoxim; pyributicarb; pyridafol; pyridate; pyriftalid; pyriminobac(-methyl) (KIH-6127); pyrimisulfan (KIH-5996); pyrithiobac(-sodium) (KIH-2031); pyroxasulfone (KIH-485); pyroxofop und dessen Ester (z.B. Propargylester); pyroxsulam; quinclorac; quinmerac; quinoclamine; quinofop und dessen Esterderivate, quizalofop und quizalofop-P und deren Esterderivate z.B. quizalofop-ethyl; quizalofop-P-tefuryl und -ethyl, renriduron; rimsulfuron (DPX-E 9636); S 275, d.h. 2-[4-Chlor-2-fluor-5-(2-propynyloxy)-phenyl]4,5,6,7-tetrahydro-2H-indazol; secbumeton; sethoxydim; siduron; simazine; simetryn; sintofen; SN 106279, d.h. 2-[[7-[2-Chlor-4-(trifluormethyl)-phenoxy]-2-naphthalenyl]-oxy]-propansäure und -methylester; sulcotrione; sulfentrazone (FMC-97285, F-6285); sulfazuron; sulfometuron(-methyl); sulfosate (ICI-A0224); sulfosulfuron; TCA(-sodium); tebutam (GCP-5544); tebuthiuron; tecnacene; tefuryltrione; tembotrione; tepraloxydim; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiafluamide, thiazafluron; thiazopyr (Mon-13200); thidiazimin (SN-24085); thidiazuron; thiencarbazone(-methyl); thifensulfuron(-methyl); thiobencarb; Ti 35; tiocarbazil; topramezone; tralkoxydim; tri-allate; triasulfuron; triaziflam; triazofenamide; tribenuron(-methyl); triclopyr; tridiphane; trietazine; trifloxysulfuron(-sodium); trifluralin; triflusulfuron und Ester (z.B. Methylester, DPX-66037); trimeturon; trinexapac; tritosulfuron; tsitodef; uniconazole; vernolate; WL 110547, d.h. 5-Phenoxy-1-[3-(trifluormethyl)-phenyl]-1H-tetrazol; D-489; ET-751; KIH-218; KIH-485; KIH-509; KPP-300; LS 82-556; NC-324; NC-330; DPX-N8189; SC-0774; DOWCO-535; DK-8910; V-53482; PP-600; MBH-001; TH-547; SYN-523; IDH-100; SYP-249; HOK-201; IR-6396; MTB-951; NC-620.

Von besonderem Interesse ist die selektive Bekämpfung von Schadpflanzen in Kulturen von Nutz- und Zierpflanzen. Obgleich die erfindungsgemäßen Salze, insbesondere die Verbindungen der Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Salze, insbesonder von Verbindungen der Formel (I) von besonderem Interesse, welche die erfindungsgemäßen Salze, insbesonder die Verbindungen der Formel (I) bzw. deren Kombinationen mit anderen Herbiziden oder Pestiziden und Safenem enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich
- R_{A}⁴: ist Wasserstoff, (C₁-C₆)-Alkyl, (C₁-C₆)-Alkoxy oder substituiertes oder unsubstituiertes Phenyl;
- R_{A}⁵: ist H, (C₁-C₈)-Alkyl, C₁-C₈(Haloalkyl), (C₁-C₄)-Alkoxy(C₁-C₈)-Alkyl, Cyano oder COOR_{A}⁹, worin R_{A}⁹ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₁₂)-Cycloalkyl oder Tri-(C₁-C₄)-alkyl-silyl ist;
- R_{A}⁶, R_{A}⁷, R_{A}⁸: sind gleich oder verschieden Wasserstoff, (C₁-C₈)-Alkyl, (C₁-C₈)-Haloalkyl, (C₃-C₁₂)-Cycloalkyl oder substituiertes oder unsubstituiertes Phenyl;
vorzugsweise:
a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", siehe Pestic. Man.), und verwandte Verbindungen, wie sie in der WO 91/07874 beschrieben sind;
b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2). 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen wie sie in EP-A-174 562 und EP-A-346 620 beschrieben sind;
d) Verbindungen vom Typ der 5-Benzyl-oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Safener sind vorzugsweise ausgewählt aus der Gruppe bestehend aus:
A) Verbindungen der Formel (S-I), 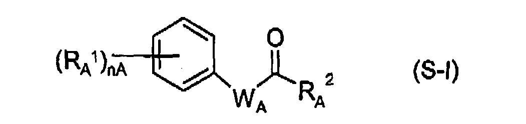 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)-Alkyl, (C₁-C₄)-Alkoxy, Nitro oder (C₁-C₄)-Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴).

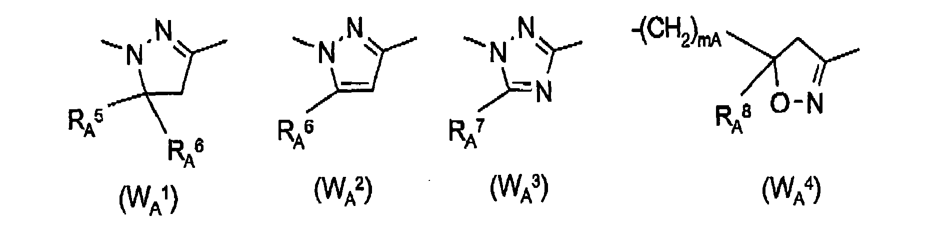

   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.
B) Chinolinderivate der Formel (S-II), 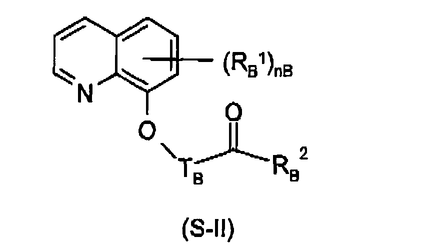 wobei die Symbole und Indizes folgende Bedeutungen haben:
   - R_{B}¹: Halogen, (C₁-C₄)-Alkyl, (C₁-C₄)-Alkoxy, Nitro oder (C₁-C₄)-Haloalkyl;
   - n_{B}: ist eine natürliche Zahl von 0 bis 5, vorzugsweise 0 bis 3;
   - R_{B}²: OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formeln OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)-Alkyl, (C₁-C₆)-Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)-Alkylresten oder mit [(C₁-C₃)-Alkoxy]-carbonyl substituiert ist;
   vorzugsweise:
   a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
   b) Verbindungen vom Typ der (6-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
C) Verbindungen der Formel (S-III) 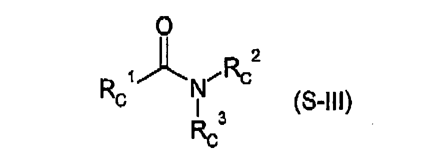 wobei die Symbole und Indizes folgende Bedeutungen haben:
   R_{C}¹ ist (C₁-C₄)-Alkyl, (C₁-C₄)-Haloalkyl, (C₂-C₄)-Alkenyl, (C₂-C₄)-Haloalkenyl, (C₃-C₇)-Cycloalkyl, vorzugsweise Dichlormethyl;
   R_{C}², R_{C}³ ist gleich oder verschieden Wasserstoff, (C₁-C₄)-Alkyl, (C₂-C₄)-Alkenyl, (C₂-C₄)-Alkinyl, (C₁-C₄)-Haloalkyl, (C₂-C₄)-Haloalkenyl, (C₁-C₄)-Alkylcarbamoyl-(C₁-C₄)-alkyl, (C₂-C₄)-Alkenylcarbamoyl-(C₁-C₄)-alkyl, (C₁-C₄)-Alkoxy-(C₁-C₄)-alkyl, Dioxolanyl-(C₁-C₄)-alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (siehe Pestic.Man.) (= N,N-Diallyl-2,2-dichloracetamid), "R-29148" (= 3-Dichloracetyl-2,2,5-trimethyl-1,3-oxazolidin von der Firma Stauffer), "R-28725" (= 3-Dichloracetyl-2,2,-dimethyl-1,3-oxazolidin von der Firma Stauffer), "Benoxacor" (siehe Pestic. Man.) (= 4-Dichloracetyl-3,4-dihydro-3-methyl-2H-1,4-benzoxazin),
   "PPG-1292" (= N-Allyl-N-[(1,3-dioxolan-2-yl)-methyl]-dichloracetamid von der Firma PPG Industries),
   "DKA-24" (= N-Allyl-N-[(allylaminocarbonyl)-methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "TI-35" (= 1-Dichloracetyl-azepan von der Firma TRI-Chemical RT)
   "Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 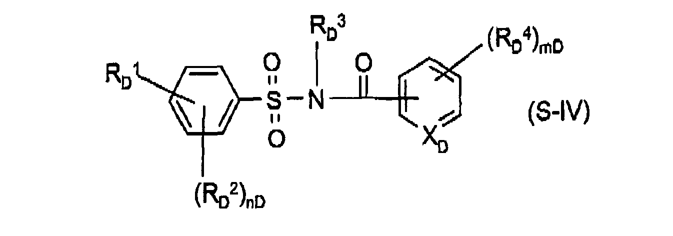 worin
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsuenyl, (C₁-C₄)-Alkoxycarbonyl oder (C₁-C₄)-Alkylcarbonyl;
   - R_{D}³: ist Wasserstoff, (C₁-C₄)-Alkyl, (C₂-C₄)-Alkenyl oder (C₂-C₄)-Alkinyl;
   - R_{D}⁴: ist Halogen, Nitro, (C₁-C₄)-Alkyl, (C₁-C₄)-Haloalkyl, (C₁-C₄)-Haloalkoxy, (C₃-C₆)-Cycloalkyl, Phenyl, (C₁-C₄)-Alkoxy, Cyano, (C₁-C₄)-Alkylthio, (C₁-C₄)-Alkyl-sulfinyl, (C₁-C₄)-Alkylsulfonyl, (C₁-C₄)-Alkoxycarbonyl oder (C₁-C₄)-Alkylcarbonyl;
   - R_{D}⁵: ist Wasserstoff, (C₁-C₆)-Alkyl, (C₃-C₆)-Cycloalkyl, (C₂-C₆)-Alkenyl, (C₂-C₆)-Alkinyl, (C₅-C₆)-Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-Alkyl und (C₁-C₄)-Haloalkyl substituiert sind;
   - R_{D}⁶: ist Wasserstoff, (C₁-C₆)-Alkyl, (C₂-C₆)-Alkenyl oder (C₂-C₆)-Alkinyl, wobei die drei letztgenannten Reste durch v_{D} Reste aus der Gruppe Halogen, Hydroxy, (C₁-C₄)-Alkyl, (C₁-C₄)-Alkoxy und (C₁-C₄)-Alkylthio substituiert sind, oder
   - R_{D}⁵: und R_{D}⁶ bilden gemeinsam mit dem dem sie tragenden Stickstoffatom einen Pyrrolidinyl- oder Piperidinyl-Rest;
   - R_{D}⁷: ist Wasserstoff, (C₁-C₄)-Alkylamino, Di-(C₁-C₄)-alkylamino. (C₁-C₆)-Alkyl, (C₃-C₆)-Cycloalkyl, wobei die 2 letztgenannten Reste durch v_{D} Substituenten aus der Gruppe Halogen, (C₁-C₄)-Alkoxy, Halogengen(C₁-C₆)-alkoxy und (C₁-C₄)-Alkylthio und im Falle cyclischer Reste auch (C₁-C₄)-Alkyl und (C₁-C₄)-Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen von Typ der N-Acylsulfonamide, z.B. der nachfolgenden Formel (S-V), die z. B. bekannt sind aus WO 97/45016 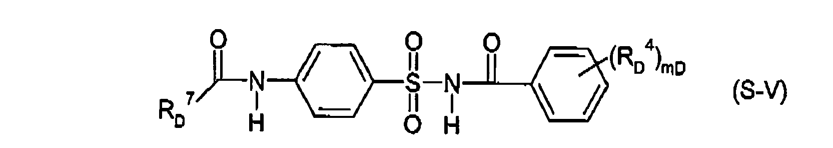 worin
   - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, Halogen-(C₁-C₆)alkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)-Alkyl, (C₁-C₄)-Alkoxy, CF₃;
   - m_{D}: 1 oder 2 bedeutet;
   - v_{D}: ist 0, 1, 2 oder 3;
   sowie
   Acylsulfamoylbenzoesäureamide, z.B. der nachfolgenden Formel (S-VI), die z.B. bekannt sind aus WO 99/16744, 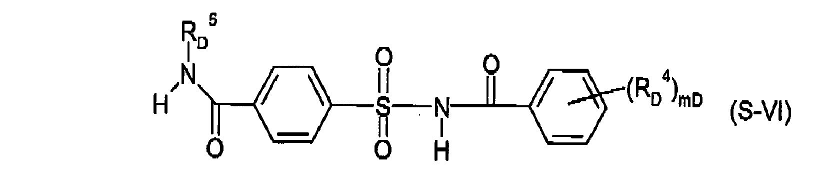 z.B. solche worin
   - R_{D}⁵ =: Cyclo-Propyl und (R_{D}⁴) = 2-OMe ist ("Cyprosulfamide", S3-1),
   - R_{D}⁵ =: Cyclo-Propyl und (R_{D}⁴) = 5-Cl-2-OMe ist (S3-2),
   - R_{D}⁵ =: Ethyl und (R_{D}⁴) = 2-OMe ist (S3-3),
   - R_{D}⁵ =: iso-Propyl und (R_{D}⁴) = 5-Cl-2-OMe ist (S3-4) und
   - R_{D}⁵ =: iso-Propyl und (R_{D}⁴) = 2-OMe ist (S3-5);
   sowie
   Verbindungen vom Typ der N-Acylsulfamoylphenylharnstoffe der Formel (S-VII), die z.B. bekannt sind aus der EP-A-365484, 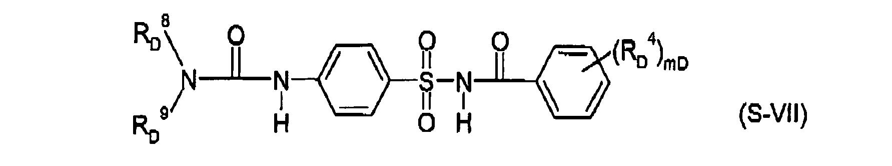 worin
   - R_{D}⁸ und R_{D}⁹: unabhängig voneinander Wasserstoff, (C₁-C₈)-Alkyl, (C₃-C₈)-Cycloalkyl, (C₃-C₈)-Alkenyl, (C₃-C₈)-Alkinyl,
   - R_{D}⁴: Halogen, (C₁-C₄)-Alkyl, (C₁-C₄)-Alkoxy, CF₃
   - m_{D}: 1 oder 2 bedeutet;
   davon insbesondere
   1-[4-(N-2-Methoxybenzoylsulfamoyl)phenyl]-3-methylhamstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylhamstoff,
   1-[4-(N-Naphthoylsulfamoyl)phenyl]-3,3-dimethylhamstoff,
G) Wirkstoffe aus der Klasse der Hydroxyaromaten und der aromatischaliphatischen Carbonsäurederivate, z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B.
   1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolylharnstoff), das als Safener für Reis gegen Schäden des Herbizids Imazosulfuron bekannt ist,
   "Cumyluron" = "JC-94.0" (= 3-(2-Chlorphenylmethyl)-1-(1-methyl-1-phenylethyl)hamstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist,
   "CSB" (= 1-Brom-4-(chlormethylsulfonyl)benzol) (CAS-Reg. Nr. 54091-06-4 von Kumiai), das als Safener gegen Schäden einiger Herbizide in Reis bekannt ist,
K) Verbindungen der Formel (S-IX),
   wie sie in der WO-A-1998/38856 beschrieben sind 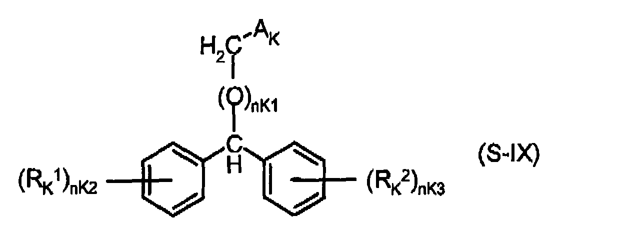 worin die Symbole und Indizes folgende Bedeutungen haben:
   - R_{K}¹, R_{K}²: unabhängig voneinander Halogen, (C₁-C₄)-Alkyl, (C₁-C₄)-Alkoxy, (C₁-C₄)-Haloalkyl, (C₁-C₄)-Alkylamino, Di-(C₁-C₄)-Alkylamino, Nitro;
   - A_{K}: COOR_{K}³ oder COOR_{K}⁴
   - R_{K}³, R_{K}⁴: unabhängig voneinander Wasserstoff, (C₁-C₄)-Alkyl, (C₂-C₆)-Alkenyl, (C₂-C₄)-Alkinyl, Cyanoalkyl, (C₁-C₄)-Haloalkyl, Phenyl, Nitrophenyl, Benzyl, Halobenzyl, Pyridinylalkyl und Alkylammonium,
   - n_{K}¹: 0 oder 1
   - n_{K}², n_{K}³: unabhängig voneinander 0,1 oder 2
   vorzugsweise: Methyl-(diphenylmethoxy)acetat (CAS-Regno: 41858-19-9),
L) Verbindungen der Formel (S-X),
   wie sie in der WO A-98/27049 beschrieben sind 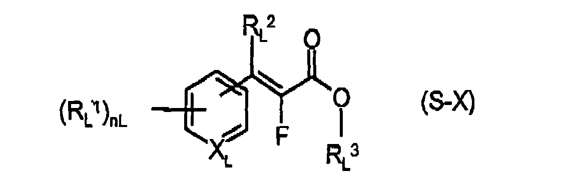 worin die Symbole und Indizes folgende Bedeutungen haben:
   - K_{L}: CH oder N,
   - n_{L}: für den Fall, dass X=N ist, eine ganze Zahl von 0 bis 4 und für den Fall, dass X=CH ist, eine ganze Zahl von 0 bis 5,
   - R_{L}¹: Halogen, (C₁-C4)-Alkyl, (C₁-C₄)-Haloalkyl, (C₁-C₄)-Alkoxy, (C₁-C₄)-Haloalkoxy, Nitro, (C₁-C₄)-Alkykhio, (C₁-C₄)-Alkylsulfonyl, (C₁-C₄)-Alkoxycarbonyl, ggf. substituiertes. Phenyl, ggf. substituiertes Phenoxy,
   - R_{L}²: Wasserstoff oder (C₁-C₄)-Alkyl
   - R_{L}³: Wasserstoff, (C₁-C₈)-Alkyl, (C₂-C₄)-Alkenyl, (₂-C₄)-Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)2-chinolone, z.B.
   1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolyl-carbonyl)-2-chinolon (CAS-Regno: 95855-00-8), wie sie in der WO-A-1999000020 beschrieben sind,
N) Verbindungen der Formeln (S-XI) oder (S-XII)
   wie sie in der WO-A-2007023719 und WO-A-2007023764 beschrieben sind 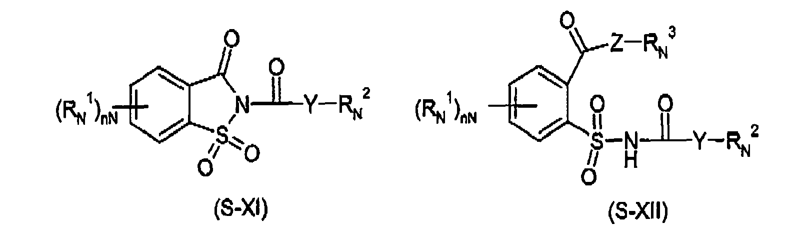 worin
   - R_{N}¹: Halogen, (C₁-C₄)-Alkyl, Methoxy, Nitro, Cyano, CF₃, OCF₃
   - Y, Z: unabhängig voneinander O oder S,
   - n_{N}: eine ganze Zahl von 0 bis 4,
   - R_{N}²: (C₁-C₁₆)-Alkyl. (C₂-C₆)Alkenyl, (C₃-C₆)-Cycloalkyl, Aryl; Benzyl, Halogenbenzyl,
   - R_{N}³: Wasserstoff, (C₁-C₆)Alkyl bedeuten;
O) eine oder mehreren Verbindungen aus Gruppe:
   1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenyrphosphorotioat (Dietholate),
   4-Carboxy-3,4-dihydro-2H-1-benzopyran-4-essigsäure (CL-304415, CAS-Regno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
einschließlich der Stereoisomeren und der in der Landwirtschaft gebräuchlichen Salze.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Einige der Safener sind bereits als Herbizide bekannt und entfalten somit neben der Herbizidwirkung bei Schadpflanzen zugleich auch Schutzwirkung bei den Kulturpflanzen.
Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10000 g/ha oder mehr Aktivsubstanz, vorzugsweise liegt sie jedoch zwischen 0,5 und 5000 g/ha, bevorzugt zwischen 0.5 und 1000 g/ha und ganz besonders bevorzugt zwischen 0.5 und 500 g/ha.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Cucumis, Cucurbita, Daucus, Glycine, Gossypium, Linum, Lycopersicon, Nicotiana, Pisum, Solanum, Vicia. Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Salze, insbesondere die Verbindungen der Formel (I), eignen sich in Abhängigkeit von der Konzentration auch zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Salze, insbesondere die Verbindungen der Formel (I), zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutem in monokotylen und dikotylen Kulturen, sowohl im Vorauflauf- als auch im Nachauflauf-Verfahren, bzw. in der sequentiellen Anwendung.

Die erfindungsgemäßen Salze, insbesondere die Verbindungen der Formel (I), zeigen einen günstigen Einfluss auf Nachfolgekulturen (Nachbauverhalten), d.h. es wurde keine oder eine extrem niedrige Phytotoxität auf verschiedene Falgekulturen, die gegenüber den erfindungsgemäßen Salzen, insbesondere den Verbindungen der Formel (I) empfindlich sind, wie beispielsweise Zuckerrübe, Sonnenblume oder Cruciferen, wie Raps, Senf und Rübsen, beobachtet.

Die Herstellung und die Verwendung der erfindungsgemäßen Wirkstoffe gehen aus den nachfolgenden Beispielen hervor.

### A. Synthesebeispiele

2-lodbenzolsuffonamid
Eine Lösung von 10 g (46.88 mmol) N-*tert*-Butylbenzolsulfonamid in 150 ml wasserfreiem Tetrahydrofuran wird bei -70°C langsam mit 61.5 ml (98.45 mmol) einer 1.6 molaren *n*-Butyllithium-Lösung in Tetrahydrofuran versetzt. Danach wird die Lösung kurz auf -30°C erwärmt und wieder auf -70°C abgekühlt, anschliessend werden 13.09 g (51.57 mmol) lod gelöst in 80 ml Tetrahydrofuran zugegeben. Nach der Zugabe wird die Lösung langsam auf Raumtemperatur erwärmt und bei dieser Temperatur 3h gerührt. Danach wird mit 50%iger wässriger Natriumthiosulfat-Lösung und mit Wasser gewaschen. Die organische Phase wird getrocknet und eingedampft. Man erhält 4.3 g (27% d. Th.) *N-tert-Butyl-2-iodbenzolsulfonamid.* 4.3 g (12.68 mmol) N-*tert*-Butyl-2-iodbenzolsulfonamid werden bei Raumtemperatur in 15 ml Trifluoressigsäure 6h gerührt. Der nach dem Eindampfen der Lösung resultierende Feststoff wird mit Wasser gewaschen. Man erhält 3.1 g (86% d. Th.) 2-lodbenzolsulfonamid.

2-lodbenzolsulfonylisocyanat
Zu einer Lösung von 5.0 g (17.66 mmol) 2-lodbenzolsuffonamid in 50 ml Xylol werden 2.1 g (21.2 mmol) n-Butylisocyanat und 0.04 g (0.35 mmol) 1,4-Diazabicyclo[2.2.2]octan zugegeben und 1 h bei 150°C (Rückfluss) gerührt. Danach wird eine Lösung von 2.27 g (11.48 mmol) Chlorameisensäuretrichlormethylester in 10 ml Xylol innerhalb von 1.5h bei 120-125°C zugetropft. Danach wird bei 150°C 1 h nachgerührt. Schliesslich werden das Lösungsmittel und alle flüchtigen Bestandteile durch Vakuumdestillation entfernt. Der Rückstand (5.0 g) wird ohne weitere Reinigung für weitere Umsetzungen verwendet.
2-lodbenzolsuffonylisoeyarlat kann mittels IR-Spektroskopie durch das Vorhandensein einer starken NCO-Schwingungsbande bei 2238 cm⁻¹ nachgewiesen werden.
1 H-NMR-Daten: (400 MHz, CDCl₃, δ, ppm): 8.19 (dd, J = 1.6, 7.6, 1H);
8.17 (dd, J = 1.6, 7.6, 1 H); 7.57 (dt, J = 1.6, 8.0, 1 H); 7.33 (dt, J = 2.0, 8.0, 1 H)

2-Iodo-N-[(4-methoxy-6-methyl-1,3,5-triazin-2-yl)carbamoyl]benzolsulfonamid
250 mg (0.88 mmol) 2-lodbenzolsulfonamid werden mit 253 mg (0.97 mmol) 2-(N-phenyloxycarbonyl)amino-4-methoe6-methyl-1,3,5-triazin in 3 ml Acetonitril vorgelegt. Dann werden 0.26 ml (1.77 mmol) 1,8-Diazabicyclo[5.4.0]undec-7-en zugesetzt, und die Lösung wird 1 h bei Raumtemperatur gerührt. Die Lösung wird dann mittles 2N Salzsäure langsam auf pH 1 eingestellt. Der ausgefallene Feststoff wird abgesaugt, mit Düsopropylether gewaschen und getrocknet. Man erhält 360 mg (90% d. Th.) 2-Iodo-N-[(4-methoxy-6-methyl-1,3,5-triazirl-2-yl)carbamoyl]benzolsulfonamid.

2-Iodo-N-[(4-methoxy-6-methyl-1,3,5-triazin-2yl)carbamoyl]benzolsulfonamid-Natriumsalz (= Verbindung I-2 der nachfolgenden Tabelle 1)
Zu einer Lösung von 5.0 g (11.13 mmol) 2-lodo-N-[(4-methoxy-6-methyl-1,3,5-triazin-2-yl)cairbamoyl]benzolsulfonamid in 500 ml Acetonitril und 50 ml Wasser werden 0.47 (11.69 mmol) Natriumhydroxid gegeben. Die Lösung wird bei Raumtemperatur über Nacht gerührt. Nach Eindampfen der Lösungsmittel und Trocknung am Hochvakuum erhält man das Natriumsalz in quantitativer Ausbeute. 1H-NMR-Daten: (300 MHz, d₆-DMSO, δ, ppm): 8.97 (br. s, 1H); 8.03 (dd, J = 1.7, 7.9, 1 H); 7.94 (dd, J = 1.1, 7.8, 1 H); 7.44 (dt, J = 1.2, 7.4, 1H); 7.10 (dt, J = 1.7, 7.4, 1 H); 3.83 (s, 3H); 2.29 (s, 3H).

In analoger Weise werden erhalten:

2-Iodo-N-[(4-methoxy-6-methyl-1,3,5-triazin-2yl)carbamoyl]benzolsulfonamid-Kaliumsalz (= Verbindung I-3 der nachfolgenden Tabelle 1) durch Reaktion mit Kaliumhydroxid.
1H-NMR-Daten: (300 MHz, d₆-DMSO, δ, ppm); 9.06 (br. s, 1H); 8.04 (dd, J = 1.6, 7.9, 1H); 7.94 (dd, J = 1.0, 7.8, 1H); 7.45 (dt, J = 1.2, 7.7, 1 H); 7.11 (dt, J = 1.5, 7.6, 1 H); 3.84 (s, 3H); 2.29 (s, 3H).

2-Iodo-N-[(4-methoxy-6-methyl-1,3,5-triazin-2yl)earbamoyl]benzolsulfonamid-Lithiumsalz (= Verbindung I-1 der nachfolgenden Tabelle 1) durch Reaktion mit Lithiumhydroxid.
1H-NMR-Daten: (300 MHz, d₆-DMSO, δ, ppm): 9.56 (br. s, 1H); 8.05 (dd, J = 1.6, 7.9, 1 H); 7.95 (dd, J = 1.1, 7.8, 1H); 7.46 (ddd, J = 1.2, 7.4, 7.8, 1 H); 7.11 (dt, J = 1.7, 7.7, 1 H); 3.86 (s, 3H); 2.30 (s, 3H).

2-Iodo-N-[(4-methoxy-6-methyl-1,3,5-triazin-2yl)carbamoyl]benzolsulfonamid-Magnesiumsalz (= Verbindung I-4 der nachfolgenden Tabelle 1) durch Reaktion mit Magnesiumhydroxid.
1H-NMR-Daten: (300 MHz, d₆-DMSO, δ, ppm): 8.96 (br, s , 1 H); 8.05 (dd, J = 1.6, 7.9, 1H); 7.94 (dd, J = 1.1, 7.8, 1 H); 7.44 (dt, J = 1.2, 7.7, 1 H); 7.10 (dt, J = 1.7, 7.5, 1 H); 3.84 (s, 3H); 2.30 (s, 3H).

2-Iodo-N-[(4-methoxy-6-methyl-1,3,5-triazin-2yl)carbamoyl]benzolsulfonamid-Kalziumsalz (= Verbindung I-5 der nachfolgenden Tabelle 1) durch Reaktion mit Kalziumhydroxid.
1H-NMR-Daten: (300 MHz, d₆-DMSO, δ, ppm): 8.87 (br. s , 1H); 8.02 (dd, J = 1.7, 7.9, 1 H); 7.93 (dd, J = 1.2, 7.8, 1 H); 7.43 (dt, J = 1.2, 7.7, 1 H); 7.08 (dt, J = 1.7, 7.6, 1 H); 3.83 (s, 3H); 2.28 (s, 3H).

2-Iodo-N-[(4-methoxy-6-methy(-1,3,5-triazin-2yl)carbamoyl]benzolsulfonamid-(2-Hydroxyeth-1-yl)ammoniumsalz (= Verbindung I-19 der nachfolgenden Tabelle 1) durch Reaktion mit 2-Aminoethanol.
1H-NMR-Daten: (300 MHz, d₆-OMSO, δ, ppm): 8.03 (dd, J =1.7, 7.9, 1 H); 7.93 (dd, J = 1.2, 7.8, 1H); 7.71 (br. s, 1H); 7.44 (dt, J = 1.2, 7.8, 1H); 7.09 (dt, J = 1.7, 7.6, 1 H); 5.11 (br, s, 1 H); 3.83 (s, 3H); 3.57 (t, J = 5.3, 2H); 2.85 (t, J = 5.5, 2H); 2.28 (s, 3H).

2-lodo-N-[(4-methoxy-6-methyl-1,3,5-triazin-2yl)carbamoyl]benzolsulfonamid-Bis-N,N-(2-hydroxyeth-1-yl)ammoniumsalz (= Verbindung I-20 der nachfolgenden Tabelle 1) durch Reaktion mit 2,2'-Iminodiethanol.
1H-NMR-Daten: (300 MHz, d₆-DMSO, δ, ppm): 8.02 (dd, J = 1.7, 7.9, 1 H); 7.93 (dd, J = 1.2, 7.8, 1H); 7.43 (dt, J = 1.2, 7.7, 1H); 7.08 (dt, J = 1.7, 7.5, 1H); 5.13 (br. s, 2H); 3.83 (s, 3H); 3.64 (t, J = 5.2, 4H); 2.98 (t, J = 5.5, 4H); 2.28 (s, 3H).

2-Iodo-N-[(4-methoxy-6-methyl-1,3,5-triazin-2yl)carbamoyl]benzolsulfonamid-Tris-N,N,N-(2-hydroxyeth-1-yl)ammoniumsalz (= Verbindung I-21 der nachfolgenden Tabelle 1) durch Reaktion mit 2,2,2'''-Nitrilotriethanol.
1 H-NMR-Daten: (300 MHz, d₆-DMSO, δ, ppm): 9.05 (br. s, 1H); 8.05 (dd, J = 1.4, 7.8, 1 H); 7.97 (br. d, J = 8.1, 1 H); 7.47 (br. t, J = 7.6, 1 H); 7.14 (br. t, J = 8.2, 1H); 4.99 (br, s, 3H); 3.86 (s, 3H); 3.65 (br. s, 6H); 3.08 (br. s, 6H); 2.31 (s, 3H).

2-Iodo-N-[(4-methoxy-6-methyl-1,3,5-triazin-2yl)carbamoyl]benzolsulfonamid-Ammoniumsalz (= Verbindung I-6 der nachfolgenden Tabelle 1) durch Reaktion mit Ammoniak.
1H-NMR-Daten: (300 MHz, d₆-DMSO, δ, ppm): 9.33 (br. s, 1 H); 8.08 (dd, J = 1.6, 7.9, 1 H); 8.00 (d, J = 7.6, 1 H); 7.50 (t, J = 7.8, 1 H); 7.18 (br. t, J = 7.3, 1 H); 7.12 (br. s, 3H); 3.87 (s, 3H); 2.34 (s, 3H).

2-Iodo-N-[(4-methoxy-6-methyl-1,3,5-triazin-2yl)carbamoyl]benzolsuifonamid-Isopropylammoniumsalz (= Verbindung I-15 der nachfolgenden Tabelle 1) durch Reaktion mit Isopropylamin.
1H-NMR-Daten: (300 MHz, d₆-DMSO, δ, ppm): 9.06 (br. s, 1 H); 8.04 (dd, J = 1.6, 7.9, 1 H); 7.95 (dd, J = 0.9, 7.7, 1H); 7.63 (br s, 3H); 7.46 (dt, J = 1.0, 7.8, 1H); 7.12 (dt, J = 1.1, 7.4, 1 H); 3.84 (s, 3H); 3.26 (spt, J = 6.6, 1 H); 2.30 (s, 3H); 1.15 (d, J = 6.5, 6H).

2-Iodo-N-[(4-methoxy-6-methyl-1,3,5-triazin-2yl)carbamoyl]benzolsulfonamid-Tetraethylammoniumsalz (= Verbindung I-12 der nachfolgenden Tabelle 1) durch Reaktion mit Tetraethylammoniumhydroxid.
1H-NMR-Daten: (300 MHz, d₆-DMSO, δ, ppm): 8.78 (br. s, 1H); 8.02 (dd, J = 1.6, 7.9, 1 H); 7.93 (dd, J = 1.1, 7.8, 1 H); 7.43 (ddd, J =1.3, 7.4, 7.8, 1H); 7.08 (ddd, J = 1.7, 7.4, 7.7, 1H); 3.82 (s, 3H); 3.19 (m, 8H); 2.27 (s, 3H); 1.15 (m, 12H).

2-Iodo-N-[(4-methoxy-6-methyl-1,3,5-triazin-2yl)carbamoyl]benzolsulfonamid-Tetrapropylammoniumsalz (= Verbindung I-14 der nachfolgenden Tabelle 1) durch Reaktion mit Tetrapropylammoniumhydroxid.
1H-NMR-Daten: (300 MHz, d₆-DMSO, δ, ppm): 8.76 (br. s, 1H); 8.02 (dd, J = 1.6, 7.9, 1H); 7.92 (dd, J =1.1, 7.8, 1H); 7.43 (ddd, J = 1.3, 7.4, 7.8, 1H); 7.08 (ddd, J = 1.7, 7.4, 7.7, 1H); 3.82 (s, 3H); 3.12 (m, 8H); 2.27 (s, 3H); 1.60 (m, 8H); 0.89 (t, J = 7.3, 12H).

2-Iodo-N-[(4-methoxy-6-methyl-1,3.5-triazin-2yl)carbamoyl]benzolsulfonamid-Tetrabutylammoniumsalz (= Verbindung I-18 der nachfolgenden Tabelle 1) durch Reaktion mit Tetrabutylammoniumhydroxid.
1H-NMR-Daten: (300 MHz, d₆-OMSO, δ, ppm): 8.76 (br, s, 1 H); 8.02 (dd, J = 1.7, 7.9, 1H); 7.92 (dd, J = 1.2, 7.8, 1 H); 7.42 (dt, J = 1.3, 7.7, 1H); 7.07 (dt, J =1.7, 7.6, 1 H); 3.82 (s, 3H); 3.16 (m, 8H); 2.26 (s, 3H); 1.57 (m, 8H); 1.31 (m, 8H); 0.93 (t, J = 7.5, 12H).

Die in der nachfolgenden Tabelle 1 beschriebenen Verbindungen werden wie direkt zuvor beschrieben oder analog zu den obigen Beispielen erhalten.

**Tabelle 1: Verbindungen der allgemeinen Formel (I), wobei durch M⁺ das jeweilige Salz der bezeichnet ist**

| | |
|---|---|
| 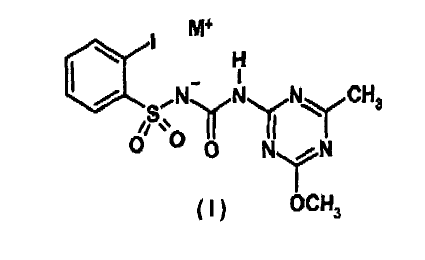 | |

| Verbindung | M⁺ |
|---|---|
| I-1 | Lithium |
| I-2 | Natrium |
| I-3 | Kalium |
| I-4 | Magnesium |
| I-5 | Calcium |
| I-6 | Ammonium |
| 1-7 | Methylammonium |
| I-8 | Dimethylammonium |
| I-9 | Tetramethylammonium |
| I-10 | Ethylammonium |
| I-11 | Diethylammonium |
| I-12 | Tetraethylammonium |
| 1-13 | Propylammonium |
| I-14 | Tetrapropylammonium |
| I-15 | Isopropylammonium |
| I-16 | Diisopropylammonium |
| I-17 | Butylammonium |
| I-18 | Tetrabutylammonium |
| I-19 | (2-Hydroxyeth-1-yl)ammonium |
| I-20 | Bis-N,N-(2-hydroxyeth-1-yl)ammonium |
| I-21 | Tris-N,N,N-(2-hydroxyeth-1-yl)ammonium |
| I-22 | 1-Phenylethylammonium |
| I-23 | 2-Phenylethylammonium |
| I-24 | Trimethyfsulfonium |
| I-25 | Trimethyloxonium |
| I-26 | Pyridinium |
| I-27 | 2-Methylpyridinium |
| I-28 | 4-Methylpyridinium |
| I-29 | 2,4-Dimethylpyridinium |
| I-30 | 2,6-Dimethylpyridinium |
| I-31 | Piperidinium |
| I-32 | Imidazolium |
| I-33 | Morpholinium |
| I-34 | 1,5-Diazabicyclo[4.3,0]non-7-enium |
| I-35 | 1,8-Diazabicyclo[5.4.0]undec-7-enium |

### B. Formulierungsbeispiele

a) Ein Stäubemittel wird erhalten, indem man 10 Gew.-Teile eines erfindungsgemäßen Salzen, insbesondere einer Verbindung der Formel (I) und 90 Gew.-Teile Talkum als Inertstoff mischt und in einer Schlagmühle zerkleinert,
b) Ein in Wasser leicht dispergierbares, benetzbares Pulver wird erhalten, indem man 25 Gewichtsteile eines erfindungsgemäßen Salzen, insbesondere einer Verbindung der Formel (I), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile eines erfindungsgemäßen Salzes, insbesondere einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man 75 Gew.-Teile einer erfindungsgemäßen Verbindung, insbesondere einer Verbindung der Formel (I), 10 Gew.-Teile ligninsulfonsaures Calcium, 5 Gew.-Teile Natriumlaurylsulfat, 3 Gew.-Teile Polyvinylalkohol und 7 Gew.-Teile Kaolin mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gew.-Teile einer erfindungsgemäßen Verbindung, insbesondere einer Verbindung der Formel (I), 5 Gew.-Teile 2,2'-dinaphthylmethan-6,6'-disulfonsaures Natrium 2 Gew.-Teile oleoylmethyltaurinsaures Natrium, 1 Gew.-Teil Polyvinylalkohol, 17 Gew.-Teile Calciumcarbonat und 50 Gew.-Teile Wasser auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wässrige Suspensionen bzw. Emulsionen mit einer Wasseraufwandmenge von umgerechnet 100 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsem und Unkräutern auf.

Beispielsweise haben die Verbindungen Nr. I-1, I**-**2, I-3, I-4, I-12, I-18, I-19 aus Tabelle 1 sehr gute herbizide Wirkung gegen Schadpflanzen wie, Matricaria inodora, Papaver rhoeas, Stellaria media, und Viola tricolor im Vorauflaufverfahren bei einer Aufwandmenge von 0.08 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 100 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf.
Beispielsweise die Verbindungen Nr. I-1, I-2, I-3, I-4, I-12, I-8, I-19 aus Tabelle 1 sehr gute herbizide Wirkung gegen Schadpflanzen wie Amaranthus retroflexus, Lolium multiflorum, Abuthilon theophrasti, Matricaria inodora, Ipomoea purpurea, Panicum minor, Stellaria media, Solanum nigrum, Veronica persica und Viola tricolor im Nachauflaufverfahren bei einer Aufwandmenge von 0.08 kg und weniger Aktivsubstanz pro Hektar.

### 3. Kufturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Verbindungen in unterschiedlichen Dosierungen besprüht. Vier bis fünf Wochen nach der Applikation und Standzeit im Gewächshaus wurde mittels optischer Bonitur festgestellt, dass efindungsgemäße Verbindungen Gramineen-Kulturen wie Gerste, Hafer, Roggen oder Weizen im Vor- und Nachauflaufverfahren selbst bei hohen Wirkstoffdosierungen ungeschädigt ließen. Die erfindungsgemäßen Salze zeigen teilweise eine hohe Selektivität und eignen sich deshalb zur Bekämpfung von unerwünschten Pflanzenwuchs in landwirtschaftlichen Kulturen.

## Patentansprüche

1. Agrochemisch wirksame Salze des 2-lodo-N-[(4-methoxy-6-methyl-1,3,5-triazin-2-yl)carbamoyl]benzolsulfanamids.

2. Agrochemisch wirksame Salze gemäß Anspruch 1 mit der allgemeinen Formel (I) 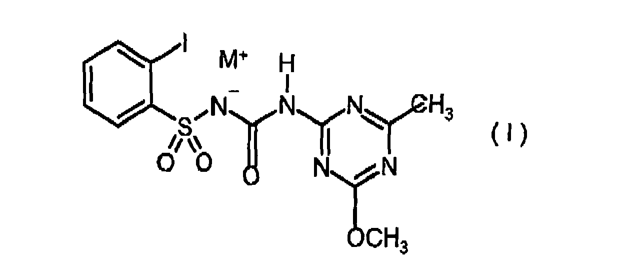 wobei das Kation (M⁺)
(a) ein lon der Alkalimetalle, bevorzugt Lithium, Natrium, Kalium, oder
(b) ein lon der Erdalkalimetalle, bevorzugt Calcium und Magnesium, oder
(c) ein lon der Übergangsmetalle, bevorzugt Mangan, Kupfer, Zink und Eisen, oder
(d) ein Ammonium-lon, bei dem gegebenenfalls ein, zwei, drei oder alle vier Wasserstoffatome, durch gleiche oder verschiedene Reste aus der Gruppe (C₁-C₄)-Alkyl, Hydroxy-(C₁-C₄)-Alkyl, (C₃-C₆)-Cycloalkyl, (C₁-C₄)-Alkoe(C₁-C₄)-alkyl, Hydroxy-(C₁-C₄)-alkoxy-(C₁-C₄)-alkyl, (C₁-C₆)-Mercaptoalkyl, Phenyl oder Benzyl substituiert sind, wobei die zuvor genannten Reste gegebenenfalls durch einen oder mehrere, gleiche oder verschiedene Reste aus der Gruppe Halogen, wie F, Cl, Br oder I, Nitro, Cyano, Azido, (C₁-C₆)-Alkyl, (C₁-C₆)-Haloalkyl, (C₃-C₆)-Cycloalkyl, (C₁-C₈)-Alkoxy, (C₁-C₆)-Haloalkoxy und Phenyl substituiert sind, und wobei jeweils zwei Substituenten am N-Atom zusammen gegebenfalls einen unsubstituierten oder substituierten Ring bilden, oder
(e) ein Phosphonium-lon, oder
(f) ein Sulfonium-lon, bevorzugt Tri-((C₁-C₄)-alkyl)-sulfonium, oder
(g) ein Oxonium-lon, bevorzugt Tri-((C₁-C₄)-alkyl)-oxonium, oder
(h) eine gegebenenfalls einfach oder mehrfach annellierte und/oder durch (C₁-C₄)-Alkyl substituierte gesättigte oder ungesättigte/aromatische N-haftige heterocyclische ionische Verbindung mit 1-10 C-Atomen im Ringsystem
ist.

3. Verbindungen gemäß Anspruch 2, **dadurch gekennzeichnet, dass** das Kation (M⁺)
(a) ein lon der Alkalimetalle, bevorzugt Lithium, Natrium, Kalium, oder
(b) ein lon der Erdalkalimetalle, bevorzugt Calcium und Magnesium, oder
(c) ein Ion der Übergangsmetalle, bevorzugt Mangan, Kupfer, Zink und Eisen, oder
(d) ein Ammonium-lon, bei dem gegebenenfalls ein, zwei, drei oder alle vier Wasserstoffatome, durch gleiche oder verschiedene Reste aus der Gruppe (C₁-C₄)-Alkyl, Hydroxy-(C₁-C₄)-Alkyl, (C₃-C₄)-Cycloalkyl, (C₁-C₂)-Alkoxy-(C₁-C₁₂)-alkyl, Hydroxy-(C₁-C₂)-alkoxy-(C₁-C₂)-alkyl, (C₁-C₂)-Mercaptoalkyl, Phenyl oder Benzyl substituiert sind, wobei die zuvor genannten Reste gegebenenfalls durch einen oder mehrere, gleiche oder verschiedene Reste aus der Gruppe Halogen, wie F, Cl, Br oder I, Nitro, Cyano, Azido, (C₁-C₂)-Alkyl, (C₁-C₂)-Holoalkyl, (C₃-C₄)-Cycloalkyl, (C₁-C₂)-Alkoxy, (C₁-C₂)-Haloalkoxy und Phenyl substituiert sind, und wobei jeweils zwei Substituenten am N-Atom zusammen gegebenfalls einen unsubstituierten oder substituierten Ring bilden, oder
(e) ein quartäres Phosphonium-lon, bevorzugt Tetra-((C₁-C₄)-alkyl)-phosphonium und Tetraphenyl-phosponium, wobei die (C₁-C₄)-Alkylreste und die Phenylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₁₂)-Haloalkoxy substituiert sind, oder
(f) ein tertiäres Suvonium-lon, bevorzugt Tri-((C₁-C₄)-alkyl)-sulfonium oder Triphenyl-sulfonium, wobei die (C₁-C₄)-Alkylreste und die Phenylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₂)-Haloalkoxy substituiert sind, oder
(g) ein tertiäres Oxonium-lon, bevorzugt Tri-((C₁-C₄)-alkyl)-oxonium, wobei die (C₁-C₄)-Alkylreste gegebenenfalls einfach oder mehrfach mit gleichen oder verschiedenen Resten aus der Gruppe Halogen, wie F, Cl, Br oder I, (C₁-C₂)-Alkyl, (C₁-C₂)-Haloalkyl, (C₃-C₄)-Cycloalkyl, (C₁-C₂)-Alkoxy und (C₁-C₂)-Haloalkoxy substituiert sind, oder
(h) ein Kation aus der Reihe der folgenden heterocyclischen Verbindungen, wie beispielsweise Pyridin, Chinolin, 2-Methylpyridin, 3-Methylpyridin, 4-Methylpyridin, 2,4-Dimethylpyridin, 2,5-Dimethylpyridin, 2,6-Dimethylpyridin, 5-Ethyl-2-methylpyridin, Piperidin, Pyrrolidin, Morpholin, Thiomorpholin, Pyrrol, Imidazol, 1,5-Diazabicyclo[4.3.0]non-5-en (DBN), 1,8-Diazabicyclo[5.4.0]undeo-7-en (DBU)
ist.

4. Verbindungen gemäß Anspruch 2, **dadurch gekennzeichnet, dass** das Kation (M⁺)
ein Natrium-lon, ein Kalium-lon, ein Lithium-lon, ein Magnesium-lon, ein Caicium-lon, ein NH₄⁺-lon, ein (2-Hydroxyeth-1-yl)ammonium-lon, Bis-N,N-(2-hydroxyeth-1-yl)ammonium-lon, Tris-N,N,N-(2-hydroxyeth-1-yl)ammonium-lon, ein Methylammonium-lon, ein Dimethylammonium-lon, ein Trimethylammonium-lon, ein Tetramethylammonium-lon ein Ethylammoniumlon, ein Diethylammonium-lon, ein Triethylammonium-lon, ein Tretraethylammonium-lon ein Isopropylammonium-lon, ein Diisopropylammonium-lon, ein Tetrapropylammonium-lon, ein Tetrabutylammonium-lon, ein 2-(2-Hydroxyeth-1-oxy)eth-9-yl-ammonium-lon, ein Di-(2-hydroxyeth-1-yl)-ammonium-lon, ein Trimethylbenzylammonium-lon, ein Tri-((C₁-C₄)-alkylrsulfonium-lon, oder ein Tri-((C₁-C₄)-alkyl)-oxonium-lon, ein Benzylammonium-lon, ein 1-Phenylethylammonium-lon, ein 2-Phenylethylammonium-lon, ein Dilsopropylethylammonium-lon, ein Pyridinium-lon, ein Piperidinium-lon, ein Imidazolium-lon, ein Morpholiniumlon, ein 1,8-Diazabicyclo[5.4.0]undee-7-enium-lon ist.

5. Verbindungen gemäß Anspruch 2, **dadurch gekennzeichnet, dass** das Kation (M⁺) ein Natrium-lon, ein Kalium-lon, ein Magnesium-lon, ein Calciumlon, oder ein NH₄⁺-lon ist.

6. Verbindungen gemäß Anspruch 2, **dadurch gekennzeichnet, dass** das Kation (M⁺) ein Natrium-lon oder ein Kalium-lon ist.

7. Verfahren zur Herstellung von Verbindungen des Anspruchs 2

8. Verfahren zum Bekämpfen von unerwünschtem Pflanzenwuchs, **dadurch gekennzeichnet, dass** man mindestens eine Verbindung gemäß einem der Ansprüche 1 bis 6 auf unwerwünschte Pflanzen und/oder ihren Lebensraum einwirken lässt.

9. Verwendung von mindestens einer Verbindung gemäß einem der Ansprüche 1 bis 6 zum Bekämpfen von unerwünschten Pflanzen.

10. Herbizides Mittel, **gekennzeichnet durch** einen Gehalt an einer Verbindung gemäß einem der Ansprüche 1 bis 6 und üblichen Streckmitteln und/oder oberflächenaktiven Mitteln.

11. 2-lodbenzolsulfonylisocyanat der Formel (VI)

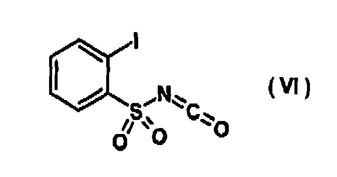

12. Verfahren zur Herstellung von 2-Iodbenzolsulfonylisocyanat, **dadurch gekennzeichnet, dass:**
Iodbenzolsulfonamid der Formel (II) 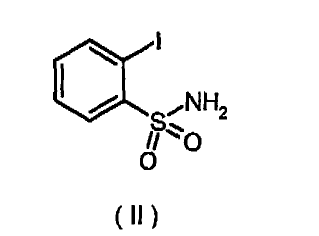
mit Phosgen, Diphosgen, oder Thiophosgen bei Temperaturen zwischen 80 °C und 150 °C umgesetzt wird.
